# EUROPEAN PATENT SPECIFICATION

(11) **EP 4 365 590 B1**
(45) Date of publication and mention of the grant of the patent: **24.09.2025**
(21) Application number: 24163286.8
(22) Date of filing: 19.03.2020
(51) Int. Cl.: A61K 47/54, A61P 35/00, A61P 43/00, C12N 15/11, C12Q 1/68, G01N 33/53, A61K 31/785, A61K 31/787, A61K 45/06, G01N 33/50, C12Q 1/6886

(54) **AGENT TARGETING DOUBLE-MEMBRANE ORGANELLE DNA**
MITTEL ZUM TARGETING VON ORGANELLEN-DNA MIT DOPPELTER MEMBRAN
AGENT CIBLANT L'ADN D'ORGANITE À DOUBLE MEMBRANE

(30) Priority: 20.03.2019 JP 2019053528
(43) Date of publication of application: 08.05.2024
(62) Divisional of application: 20773944.2
(73) Proprietor: Juntendo Educational Foundation, Tokyo 113-8421 (JP)
(72) Inventor: Nagase, Hiroki, Chiba, 260-8717 (JP); Watanabe, Takayoshi, Chiba, 260-8717 (JP); Koshikawa, Nobuko, Chiba, 260-8717 (JP); Yasui, Nanami, Chiba, 260-8717 (JP)
(74) Representative: J A Kemp LLP

(56) References cited:
- WO-A1-2012/133896
- HIDAKA TAKUYA ET AL: "Creation of a Synthetic Ligand for Mitochondrial DNA Sequence Recognition and Promoter-Specific Transcription Suppression", JOURNAL OF THE AMERICAN CHEMICAL SOCIETY, vol. 139, no. 25, 28 June 2017 (2017-06-28), pages 8444 - 8447, XP055909890, ISSN: 0002-7863, DOI: 10.1021/jacs.7b05230
- JACEK ZIELONKA ET AL: "Mitochondria-Targeted Triphenylphosphonium-Based Compounds: Syntheses, Mechanisms of Action, and Therapeutic and Diagnostic Applications", CHEMICAL REVIEWS, vol. 117, no. 15, 27 June 2017 (2017-06-27), US, pages 10043 - 10120, XP055639154, ISSN: 0009-2665, DOI: 10.1021/acs.chemrev.7b00042
- KOSHIKAWA ET AL: "Study of mitochondria-associated diseases by pathogenic mitochondrial DNA replication delay", 25 July 2014 (2014-07-25), XP093035525, Retrieved from the Internet <URL:https://kaken.nii.ac.jp/ja/file/KAKENHI-PROJECT-25461573/25461573seika.pdf> [retrieved on 20230330]
- HERRNSTADT CORINNA ET AL: "A high frequency of mtDNA polymorphisms in HeLa cell sublines", MUTATION RESEARCH, vol. 501, no. 1-2, 16 February 2002 (2002-02-16), AMSTERDAM, NL, pages 19 - 28, XP093173266, ISSN: 0027-5107, DOI: 10.1016/S0027-5107(01)00304-9
- YU ZUTAO ET AL: "Therapeutic gene regulation using pyrrole-imidazole polyamides", ADVANCED DRUG DELIVERY REVIEWS, ELSEVIER, AMSTERDAM , NL, vol. 147, 10 February 2019 (2019-02-10), pages 66 - 85, XP085927736, ISSN: 0169-409X, [retrieved on 20190210], DOI: 10.1016/J.ADDR.2019.02.001
- KOSHIKAWA NOBUKO ET AL: "A PI polyamide-TPP conjugate targeting a mtDNA mutation induces cell death of cancer cells with the mutation", CANCER SCIENCE, vol. 112, no. 6, 2 April 2021 (2021-04-02), JP, pages 2504 - 2512, XP093172846, ISSN: 1347-9032, DOI: 10.1111/cas.14912

## Description

### Technical Field

The present invention relates to a conjugate of a low-molecular-weight compound targeting double-membrane organelle DNA.

### Background Art

Lipophilic cations and the like have been developed as techniques of delivering drugs to double-membrane organelles such as mitochondria or chloroplasts (Non Patent Literature 1), and their application to the treatment of mitochondria-related disease has been attempted (Patent Literatures 2 to 5). Meanwhile, gene mutations in mitochondrial DNA have also been confirmed to accumulate at lesional sites of not only mitochondrial disease but also lifestyle-related disease or cancer. It has also been reported that the transcription of mitochondrial gene can be regulated by targeting a mitochondrial DNA sequence (Non Patent Literature 2). The possibility has also been reported that hairpin pyrrole-imidazole polyamide (PIP) targets a pathogenic mitochondrial DNA sequence and can decrease the number of mitochondria having a pathogenic gene sequence (Patent Literature 1). However, any method for efficiently inducing biological activity for cells having mutant mitochondrial DNA has not yet been established.

Mitochondrial disease often has a pathogenic mutation in mitochondrial DNA and develops by increase in copy number thereof. It has also been reported that various diseases such as lifestyle-related disease develop by increase in the number of cells having a pathogenic mutation in mitochondrial DNA. Many cases have been further reported in which cancer cells have a mutation in mitochondrial DNA and this mutation is in homoplasmy (state in which every mitochondrial DNA is replaced with mutant mitochondrial DNA) (Non Patent Literature 3). Any treatment method directly targeting a mitochondrial DNA mutation has not been developed for pathological conditions having such a pathogenic mutation in mitochondrial DNA.

The present inventors have diligently conducted search for and research on compounds that recognize a mutation in mitochondrial DNA, are retained in mitochondria for a long period, and cause biological activity for cells having a specific mutation in mitochondrial DNA. As a result, the present inventors have found that a conjugate in which a mitochondrion penetrating compound and a low-molecular-weight linear compound that binds to a DNA minor groove are attached via a linking moiety highly efficiently imparts biological activity to cells having a specific mitochondrial DNA sequence, completing the present invention. The possibility has been further reported that a lipophilic cation conjugate influences plant cells and can also penetrate chloroplasts (Non Patent Literatures 4 and 5). Although it had not been revealed that a conjugate of a low-molecular-weight compound targeting double-membrane organelle DNA can actually penetrate live cells of plant roots or leaves, the present inventors have been able to confirm this, demonstrating the possibility of application to plants.

### Citation List

### Patent Literature

Patent Literature 1: International Publication No. WO2012/133896
Patent Literature 2: International Publication No. WO2011/150494
Patent Literature 3: JP Patent Publication (Kohyo) No. 2011-501731 A (2011)
Patent Literature 4: JP Patent Publication (Kohyo) No. 2016-523926 A (2016)
Patent Literature 5: International Publication No. CN2008/801154230

### Non Patent Literature

Non Patent Literature 1: Chem Rev. 2017 117 (15) 10043-10120
Non Patent Literature 2: J. Am. Chem. Soc., 2017, 139 (25) 8444-8447
Non Patent Literature 3: Sci Rep. 2017 Nov 14; 7(1): 15535
Non Patent Literature 4: Plant Physiol. (1983) 73, 169-174
Non Patent Literature 5: Mitochondrion (2019) 46, 164-171 Available online May 01 2018

### Summary of Invention

### Technical Problem

### Mitochondrial DNA mutation-related lesion

Mitochondria are present in higher organisms including plants. Human mitochondrial DNA has a B-DNA double helix structure and encodes 37 genes, 13 of which encode respiratory chain complex subunit I, III, IV or V as structure genes and participate in the activation of respiratory chains, ATP production, and the production of reactive oxygen species. DNA mutations therein are related to various pathological conditions. One cell has several thousands of mitochondria and also has several thousands of copies of mitochondrial DNA, in which DNA mutations frequently observed. A given copy number or more of mutated pathogenic mitochondrial DNA induces mitochondrial dysfunction and participates in diseases such as mitochondrial disease, aging, lifestyle-related disease, and cancer. Particularly, a phenomenon, called homoplasmy, in which every mitochondrial DNA has a mutation, has also been reported for cancer.

### Compound targeting mitochondrion

The development of various compounds targeting mitochondria has been attempted in order to treat pathological conditions related to mitochondria. However, any radical treatment method targeting mutant mitochondrial DNA has not yet been developed. As for drug delivery to mitochondria, a technique of delivering compounds by lipophilic cations through the use of plasma membrane potential difference and mitochondrial double-membrane potential has been developed, and such compounds have been reported to accumulate at 100 to 500 times their treatment concentrations in mitochondrial matrix.

### DNA minor groove binding compound

Antibiotics produced by actinomycetes or the like are known to include compounds that recognize DNA minor grooves in a sequence-specific manner. Hairpin PI polyamide that recognizes double-stranded B-DNA has been studied by the application of this sequence recognition mechanism, and its delivery into the nuclei and into mitochondria has been confirmed, suggesting the possibility that this compound can be used in disease treatment by binding to nuclear DNA or mitochondrial DNA and influencing a genomic structure or the like. It has been reported that hairpin PI polyamide and some linear DNA binding compounds such as distamycin are delivered into mitochondria, not into the nuclei. However, it has been reported that these compounds cannot be retained therein for a long period and are translocated to ER or Golgi body, suggesting that the compounds cannot exhibit long-term bioactivity in mitochondria, which has also been confirmed by the inventors (Bioorganic & Medicinal Chemistry Letters 11 (2001) 769-772).

### Conjugate of compound targeting mitochondrion and hairpin pyrrole-imidazole polyamide (PIP)

It has been reported that a conjugate of a mitochondria penetrating peptide (MPP) which targets mitochondria, and a hairpin pyrrole-imidazole polyamide compound which targets mitochondrial DNA is retained in mitochondria and can regulate the expression of mitochondrial gene. The present inventors have also delivered hairpin pyrrole-imidazole polyamide (PIP) using a lipophilic cation triphenylphosphonium (TPP), and reported the tendency to decrease the copy number of mutant mitochondrial DNA, albeit not statistically significantly. However, the hairpin PIP is not always appropriate for drug development because of its large molecular weight and complicated synthesis process.

### Solution to Problem

In the present invention, it has been found through diligent effort to develop more effective and more appropriate drug candidates for mitochondrial DNA mutations that a conjugate in which a linear compound that recognizes a sequence and binds to a DNA minor groove is attached to a lipophilic cation can decrease the number of synthesis reaction steps and can halve a molecular weight, as compared with a conjugate of hairpin PI polyamide, and furthermore, can induce mitochondria specific autophagy (mitophagy), increase a mitochondrial DNA copy number, and decrease a mutant mitochondrial DNA copy number, in cells having a mitochondrial DNA mutation, and can induce cell death in cells having the homoplasmy of mutant mitochondrial DNA or a large copy number thereof. This has revealed that a compound that is more suitable for drug development and effectively produces bioactivity can be synthesized according to the present invention, completing the present invention. It has been further confirmed that cells resistant to cell death are present and the cell death of these resistant cells can be induced by the administration of a cell death suppressor inhibitor or the like. Moreover, effects on organisms having cell walls, such as plants, are also expected from the confirmed delivery into live cells of plant roots or leaves.

Specifically, the present invention is as follows.

A conjugate of a DNA binding compound that specifically binds to a sequence of double-membrane organelle DNA, and a double-membrane organelle localizable compound, wherein the conjugate has a structure represented by the following formula (X), (XXII) or (XXIII): 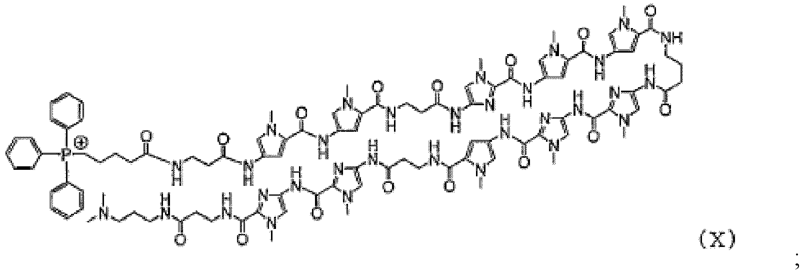 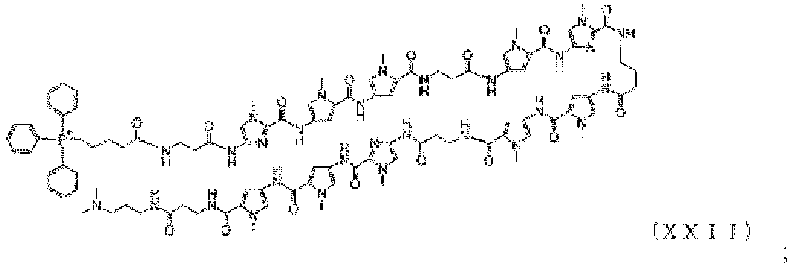 or

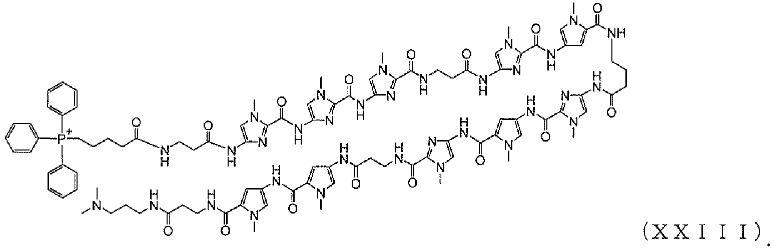

A conjugate of a DNA binding compound that specifically binds to a sequence of double-membrane organelle DNA, and a double-membrane organelle localizable compound, wherein the DNA binding compound has a structure represented by the following formula (VII) and the double-membrane organelle localized compound is TPP (triphenylphosphonium), wherein the compound of formula (VII) and the TPP are attached directly or via a linker:

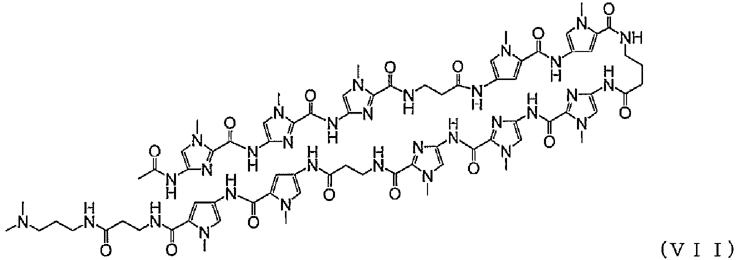

Also provided is a composition that binds to a mitochondrial disease-related mitochondrial DNA sequence, comprising a conjugate of the invention.

Further provided is a pharmaceutical composition comprising a conjugate according to the invention.

Additionally provided is the conjugate of the invention, or the pharmaceutical composition of the invention, which is further combined with a cytocidal drug.

Preferably, the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5-dichloro-3-(4-methyl-1-piperazinyl)-6-[2-(1-piperidinyl)-1,3-thiazol-5-yl]benzo-1,4-quinone, Nutlin-3, MI-63, and any derivative thereof.

Also provided is the conjugate of the invention or the pharmaceutical composition of the invention for use in treatment of mitochondrial disease.

In one embodiment, the mitochondrial disease is selected from the group consisting of chronic progressive external ophthalmoplegia, myoclonic epilepsy and ragged-red fiber disease, mitochondrial encephalomyopathy, lactic acidosis, and stroke-like episodes (MELAS), and Leigh syndrome.

Also provided is the conjugate of the invention or the pharmaceutical composition of the invention for use in treatment of cancer.

In one embodiment, the cancer is selected from the group consisting of pancreatic cancer, colorectal cancer, thyroid gland cancer, lung cancer, head and neck cancer, uterine cervical cancer, endometrial cancer, ovary cancer, myelodysplastic syndrome, adenocarcinoma of the colon, and neuroblastoma.

Further provided is a product which is:
(a) a kit comprising a conjugate of the invention;
(b) a research reagent kit comprising a conjugate of the invention;
(c) a kit for treatment comprising a conjugate of the invention; or
(d) a kit for diagnosis comprising a conjugate of the invention.

Also provided is a kit comprising a pharmaceutical composition of the invention and a cytocidal drug in combination.

Preferably, the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5-dichloro-3-(4-methyl-1-piperazinyl)-6-[2-(1-piperidinyl)-1,3-thiazol-5-yl]benzo-1,4-quinone, and any derivative thereof.

### Advantageous Effects of Invention

The present invention provides a conjugate that can bind to a double-membrane organelle (e.g., mitochondrion) DNA sequence and modify a double-membrane organelle (e.g., mitochondrion) function. According to a preferred aspect of the present invention, the conjugate of the present invention can be used as a pharmaceutical composition or an anti-mitochondrial disease agent, an anti-lifestyle-related disease agent, an anticancer agent, an anti-senescence agent, an antibiotic, etc.

The conjugate of the present invention recognizes a mitochondrial DNA sequence and is considered to thereby trigger the replication and transcription of mitochondrial DNA, mitochondria specific autophagy (mitophagy), mitochondrial activation and/or increase or decrease in mitochondrial DNA copy number by self-control for mitochondrial autophagy, and the induction of cell death or cellular senescence in the homoplasmy of mutant mitochondrial DNA or a state close thereto. It is readily conceivable that through these phenomena, the conjugate of the present invention develops various forms that can regulate the functions of mitochondria or the like in eukaryotes including animals or plants having double-membrane organelles such as mitochondria, living cells and experimental systems.

Specifically, the present invention provides a technique of delivering a low-molecular-weight compound that binds to a specific genomic sequence of mitochondria or the like to mitochondria or the like and a design or synthesis method therefor. The application of this technique permits more convenient and sequential design or synthesis of therapeutic drugs for mitochondria-related disease that can specifically recognize a DNA mutant sequence of a pathogenic or unhealthy mitochondrion. Existing therapeutic drugs, foods with functional claims, or foods for specified health use for mitochondrial disease merely provide symptomatic prevention or treatment, whereas it can be readily expected that the conjugate according to the present invention can provide a compound that exhibits a radical prophylactic or therapeutic effect. The conjugate of present invention is further applied to cell therapy or a technique of delivery to normal mitochondria and thereby presumably used for a wide range of purposes such as applicability to quality control by the diagnosis and removal of unhealthy mitochondria or unhealthy cells having mutant mitochondrial DNA.

The conjugate of the present invention induces cell death in cells having a large mutant mitochondrial DNA copy number. On the other hand, the conjugate of the present invention enables a novel revolutionary therapeutic drug to be developed as an ideal therapeutic drug that decreases the number of mutant mitochondrial DNA while minimizing influence on cells or living bodies, by gradually decreasing the copy number of mutant mitochondrial DNA and promoting increase in or activation of the total number of mitochondria at the same time with the decrease in the number of mutant mitochondrial DNA, when the ratio of normal mitochondrial DNA to mutant mitochondrial DNA is at or above a given level. The conjugate of the present invention is expected to pursue research and development toward the initiation of clinical trials (physical property tests, safety tests, pharmacokinetics, and large-scale synthesis in GMP) in the future. It is conceivable that the application of the method of the present invention permits development of a further revolutionary therapeutic drug that exerts a similar, additive or synergistic effect.

Although the conjugate of the present invention induces cell death in cells having a large mutant mitochondrial DNA copy number, the presence of cells resistant to the cell death and senescence-like change have been confirmed. Research on senolytic drugs is ongoing in recent anti-senescence research, and the clinical application of inhibitors such as BCL2, BCL-XL, BCL-W, and MDM2 effective for the cell death induction of senescent cells has been attempted. It is conceivable that the conjugate of the present invention can induce cellular senescence-like cells and permits development of further revolutionary add-on therapy or the like by exerting a synthetic lethal effect that potentiates the senolytic therapeutic effect through combined use with treatment with the senolytic drug.

The senolytic drug will be mentioned later.

The conjugate of the present invention permits shortening of its DNA minor groove recognition site because of the mitochondrial genome as small as 16,569 base pairs, and has been confirmed to be effective even with a linear-chain compound having a small molecular weight and confirmed to have skin penetrability. It is conceivable that the method of the present invention combined with transdermal absorption formulation can expand administration routes and also permits development of a revolutionary treatment method such as a local therapeutic drug or prophylactic drug for a local lesion or senescence by the potentiation of a local therapeutic effect.

### Brief Description of Drawings

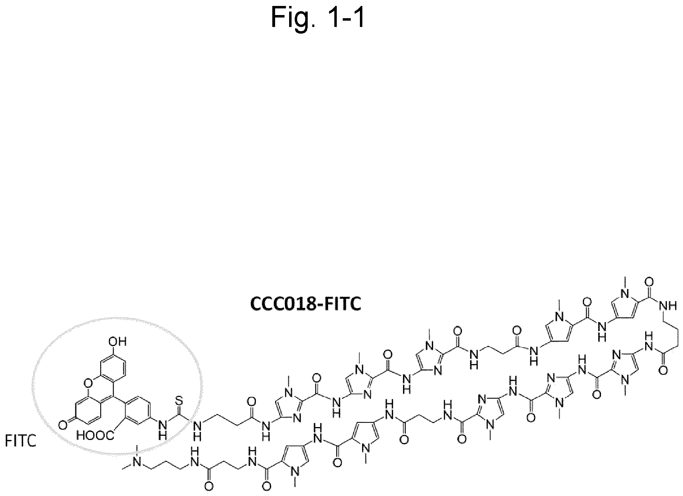

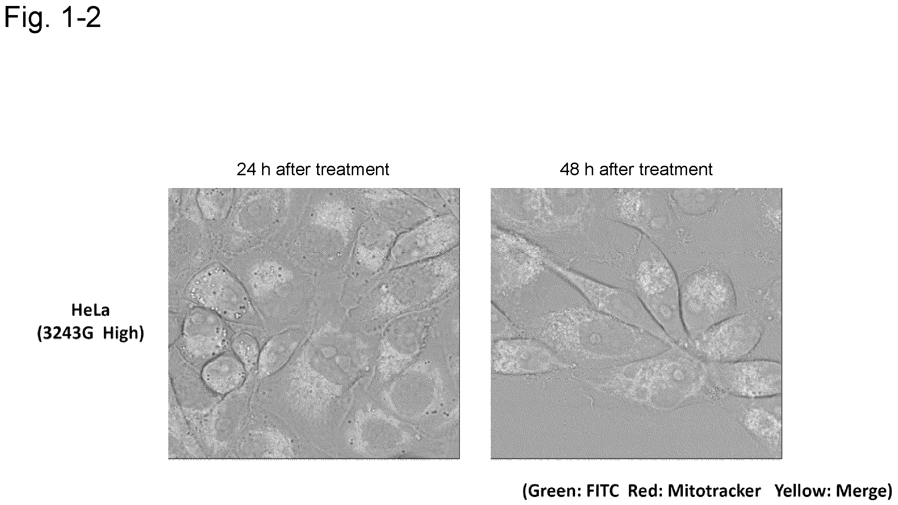

[Figure 1-1] Figure 1-1 is a diagram showing the chemical structure of FITC-labeled hairpin polyamide (CCC0018-FITC) targeting mitochondrial DNA A3243G mutation of MELAS.
[Figure 1-2] Figure 1-2 is a diagram showing the time-dependent intracellular localization of FITC-labeled hairpin polyamide (CCC0018-FITC) targeting mitochondrial DNA A3243G mutation of MELAS.
[Figure 2] Figure 2 is a diagram showing the mitochondrial localization of a pyridinium-indole-TPP conjugate. Figure 2A shows the colocalization of the pyridinium-indole-TPP conjugate with mitochondria. Figure 2B shows mitochondrial non-colocalization of pyridinium-indole.
[Figure 3] Figure 3 is a diagram showing the synthesis scheme of a conjugate of hairpin polyamide and TPP (CCC019-TPP) targeting mitochondrial DNA A3243G mutation of MELAS, a structure thereof and HPLC and mass spectrometry results.
[Figure 4] Figure 4 is a diagram showing the colocalization of CCC019-TPP with mitochondria.
[Figure 5] Figure 5 is a diagram showing results in which the total mitochondrial DNA copy number was increased in an experiment of CCC019-TPP administration to HeLa (3243G Low) cybrids. Figure 5A shows results obtained 48 days later, and Figure 5B shows results obtained 63 days later.
[Figure 6] Figure 6 is a diagram showing results in which the ratio of a normal mitochondrial DNA copy number to a mutant mitochondrial copy number was increased in an experiment of CCC019-TPP administration to HeLa (3243G Low) cells. Figure 6A shows results obtained 48 days later, and Figure 6B shows results obtained 63 days later.
[Figure 7] Figure 7 is a diagram showing results in which mitophagy (LC3-positive) indicating mitochondrial autophagy was induced in an experiment of CCC018-TPP administration to HeLa (3243G High) cells and HeEB 1 cells.
[Figure 8] Figure 8 is a diagram showing the structure of a compound CCCh531-TPP with a decacyclic structure.
[Figure 9] Figure 9 is a diagram showing results of high-performance liquid chromatography analysis (A) and mass spectrometry (B) of CCCh531-TPP.
[Figure 10] Figure 10 is a diagram showing the suppression of proliferation of HeLa cells (A) and C33A (B) by CCCh531-TPP.
[Figure 11] Figure 11 is a diagram showing the structure of a compound CCCh560-TPP with a decacyclic structure.
[Figure 12] Figure 12 is a diagram showing results of high-performance liquid chromatography analysis (A) and mass spectrometry (B) of CCCh560-TPP.
[Figure 13] Figure 13 is a diagram showing the suppression of proliferation of C33A (A), HeLa cells (B), HDF (C), Siha (D), Caski (E) and ME180 (F) by CCCh560-TPP
[Figure 14-1] Figure 14-1 is a diagram showing typical senolytic drugs (part 1).
[Figure 14-2] Figure 14-2 is a diagram showing typical senolytic drugs (part 2).
[Figure 14-3] Figure 14-3 is a diagram showing typical senolytic drugs (part 3).
[Figure 14-4] Figure 14-4 is a diagram showing typical senolytic drugs (part 4).
[Figure 14-5] Figure 14-5 is a diagram showing typical senolytic drugs (part 5).
[Figure 14-6] Figure 14-6 is a diagram showing typical senolytic drugs (part 6).
[Figure 14-7] Figure 14-7 is a diagram showing typical senolytic drugs (part 7).
[Figure 14-8] Figure 14-8 is a diagram showing typical senolytic drugs (part 8).
[Figure 14-9] Figure 14-9 is a diagram showing typical senolytic drugs (part 9).

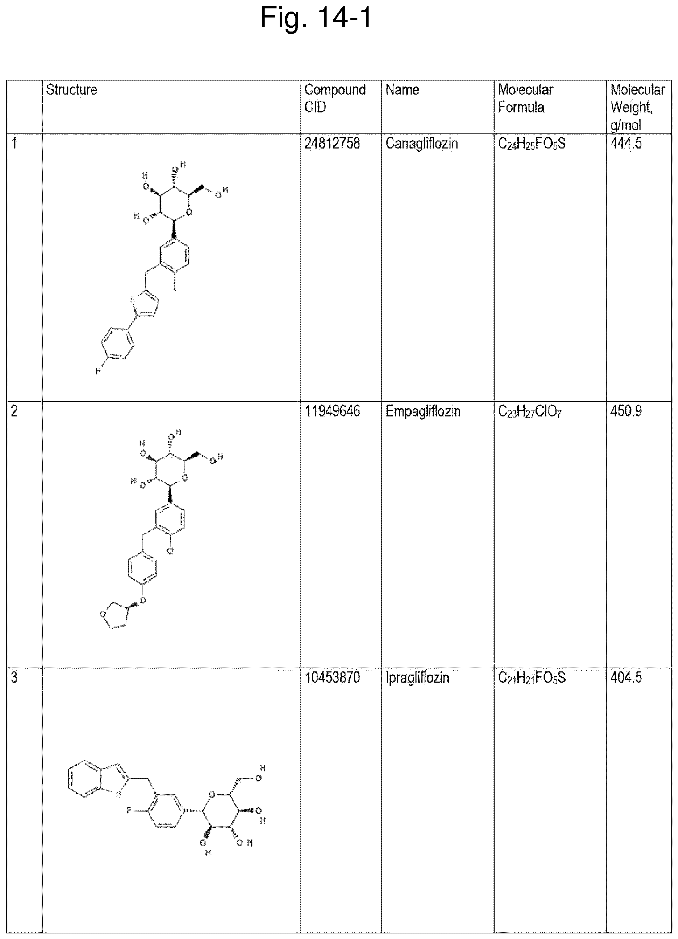

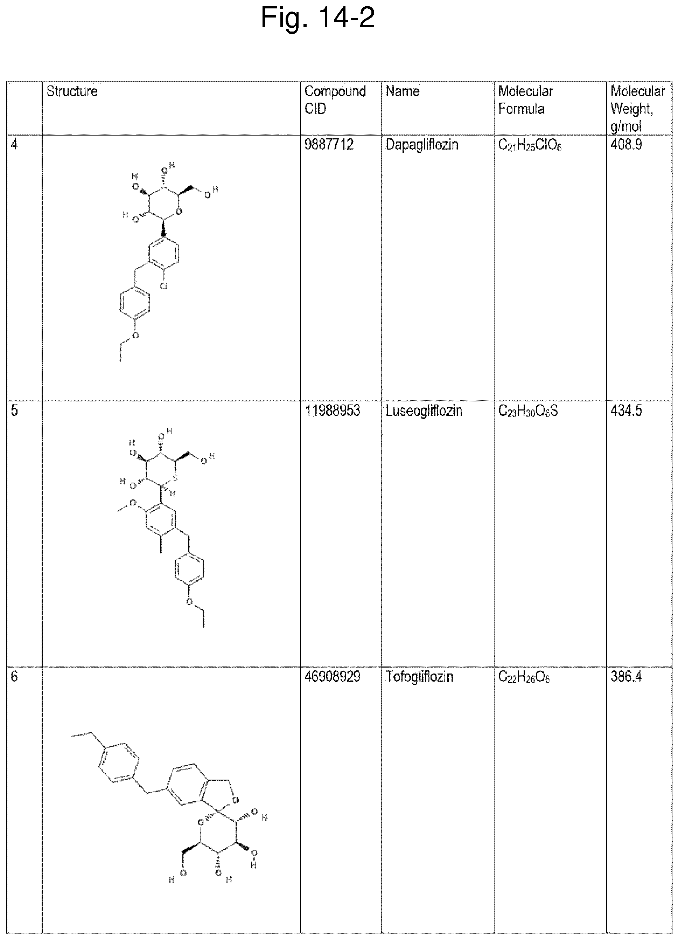

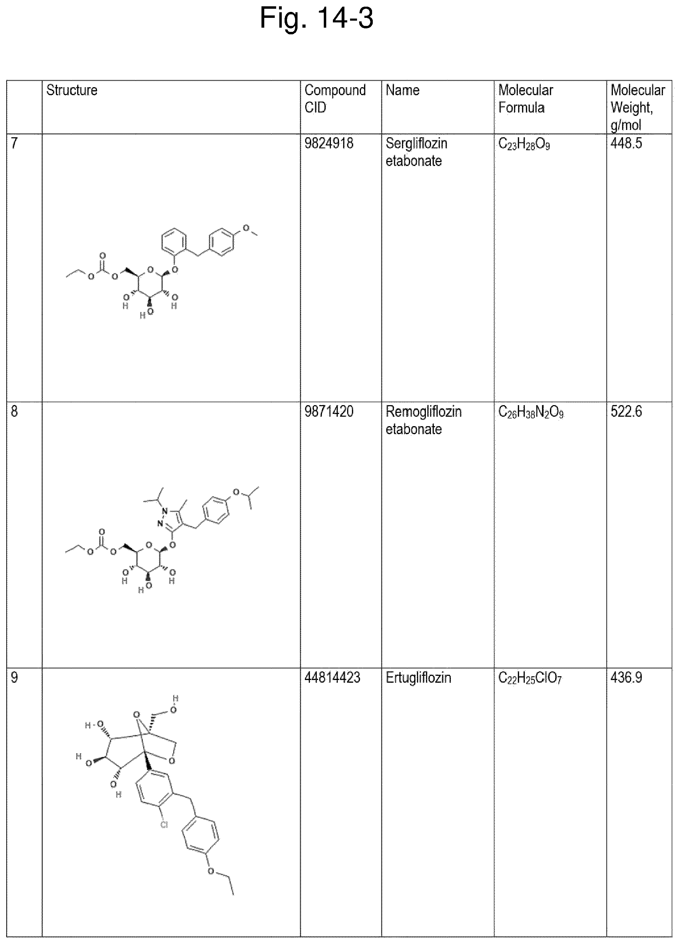

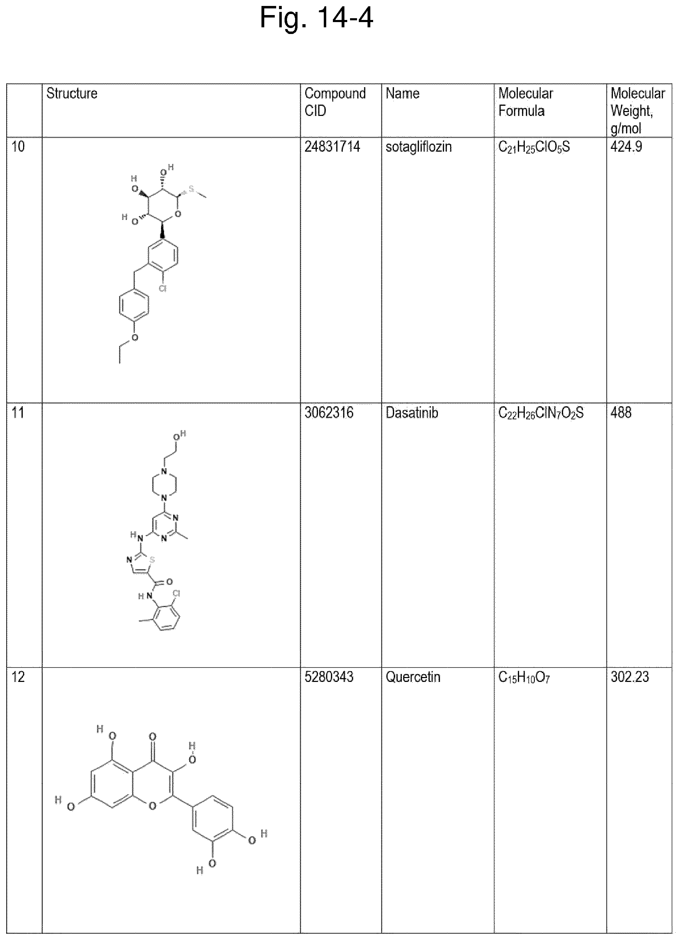

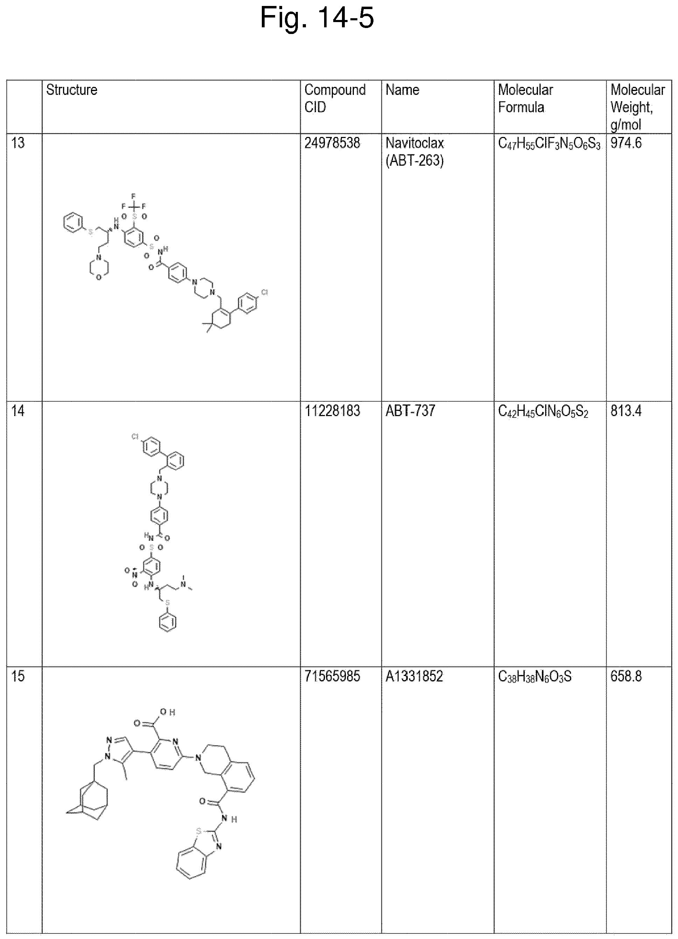

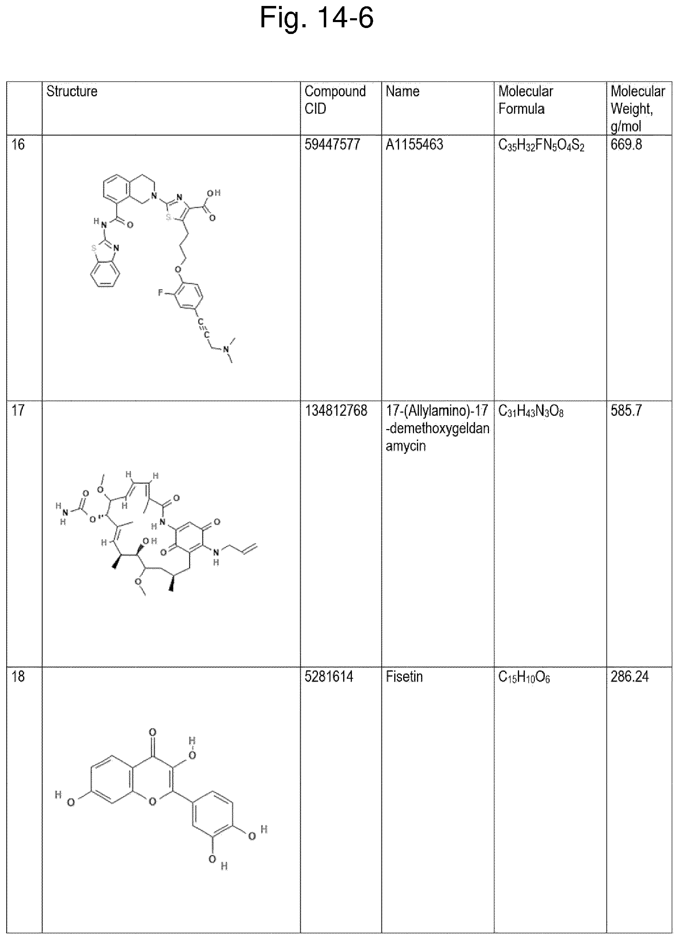

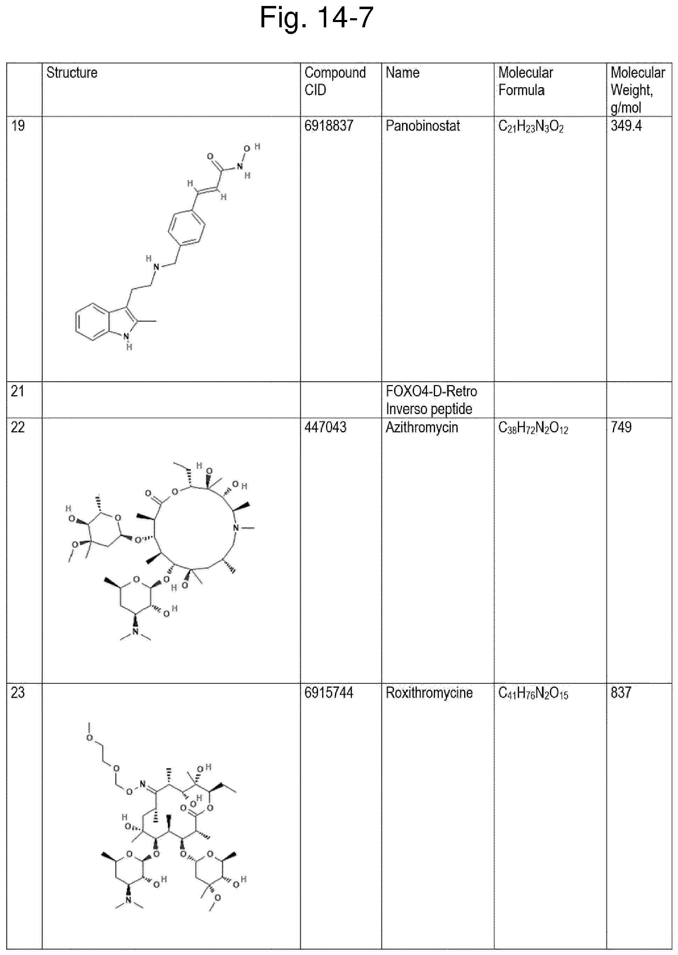

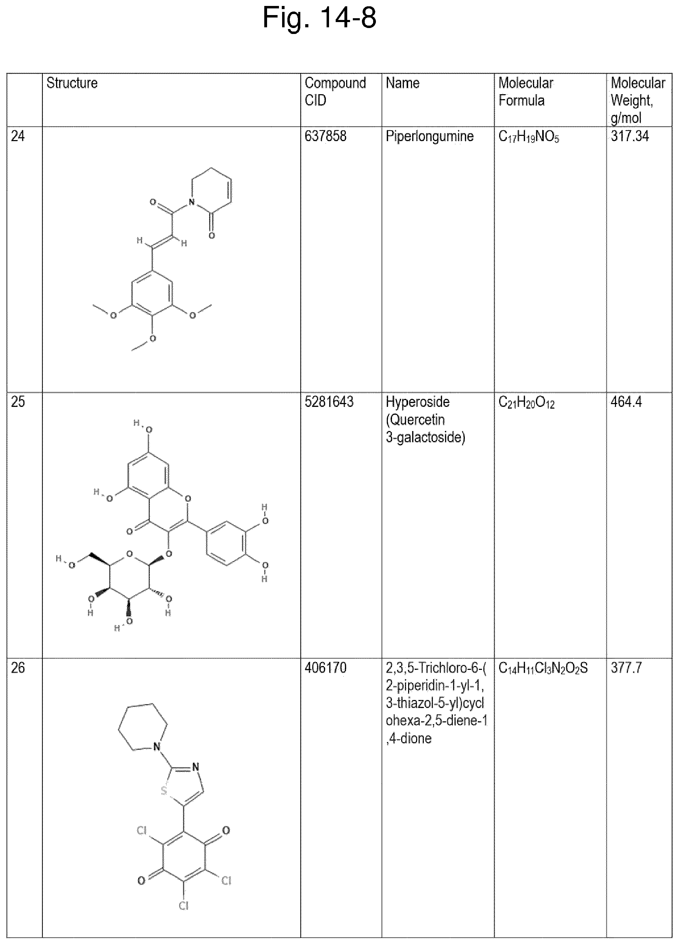

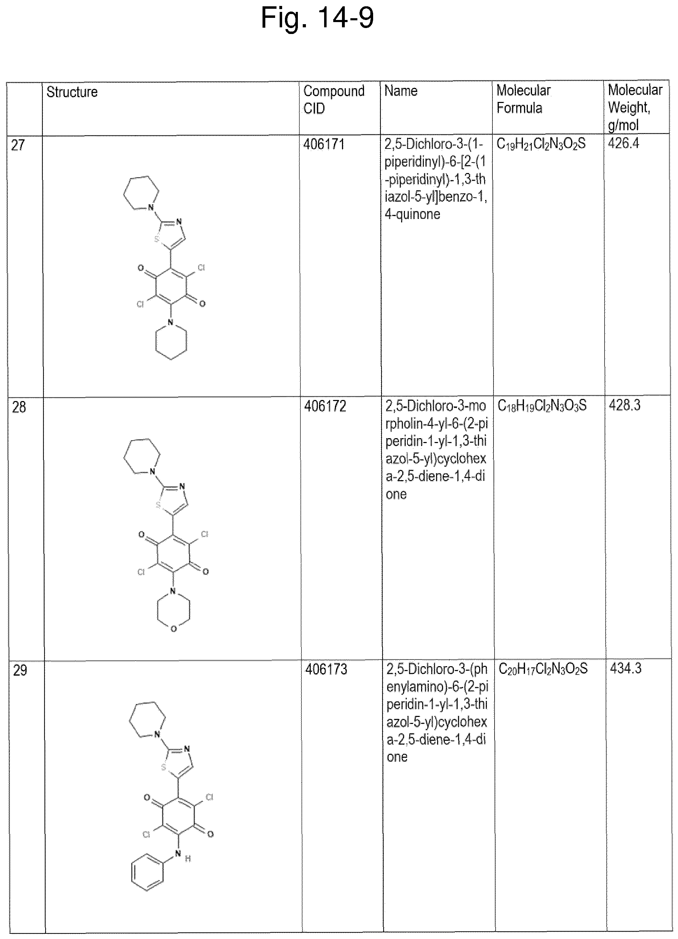

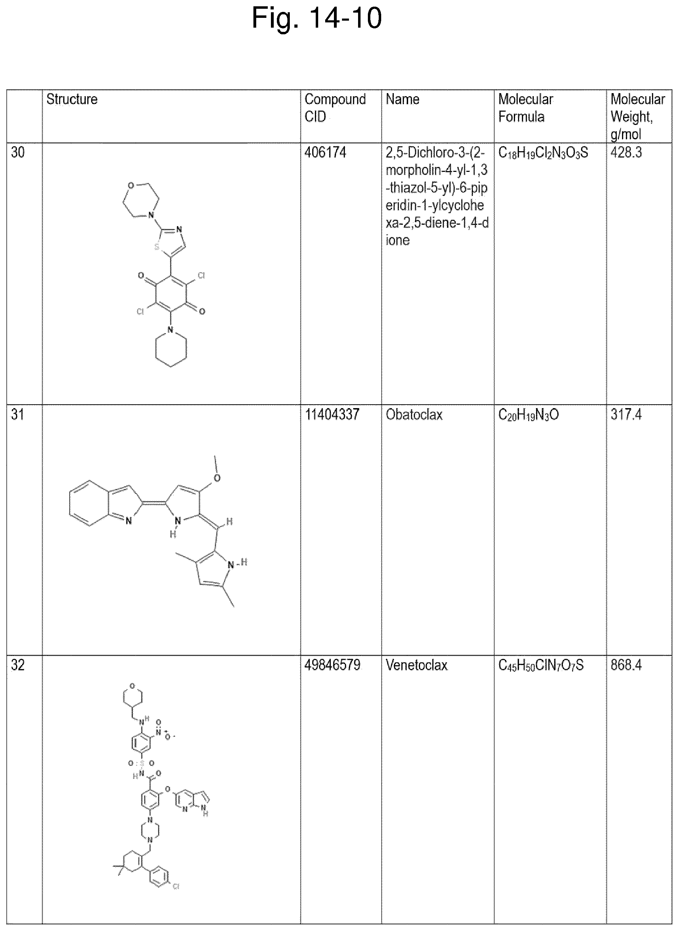

[Figure 14-10] Figure 14-10 is a diagram showing typical senolytic drugs (part 10).

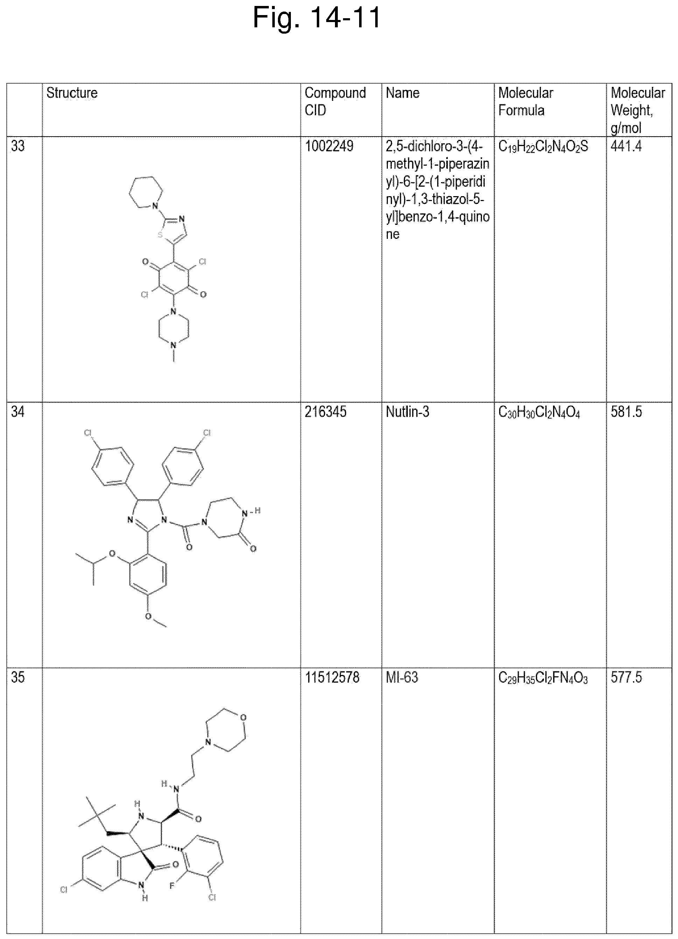

[Figure 14-11] Figure 14-11 is a diagram showing typical senolytic drugs (part 11).

### Description of Embodiments

Hereinafter, the present invention will be described in detail.

The present invention provides a conjugate of a DNA binding compound that binds to a specific sequence of DNA of a double-membrane organelle such as a mitochondrion, and a double-membrane organelle localizable compound, and design and a synthesis method therefor. This conjugate acts on DNA of a double-membrane organelle such as a mitochondrion and thereby permits a method for preventing, treating, or diagnosing a pathological condition related to a double-membrane organelle such as a mitochondrion, for example, by decrease in the copy number of double-membrane organelles such as mitochondria having a specific sequence (e.g., change in a function of a double-membrane organelle) or the induction of cell death through the dysfunction of a double-membrane organelle such as a mitochondrion.

The double-membrane organelle includes a mitochondrion as well as a chloroplast and the like.

Examples of the specific sequence of DNA of a double-membrane organelle include mitochondrial DNA sequences, mitochondrial disease pathogenic mutant DNA sequences, mitochondria-related disease mutant DNA sequences, sequences with a larger DNA copy number in lesional cells having a mitochondrial DNA polymorphism than that in normal cells, and double-membrane organelle DNA sequences registered in gene databases.

Mitochondrial DNA is derived from a mother and is present at several hundreds to several thousands of copies per cell. Since the mitochondrial DNA is localized in cytoplasmic organelles lacking a defensive mechanism as found in nuclear membranes, mutations occur highly frequently due to reactive oxygen species (ROS) as an internal factor or carcinogens, radiation, etc. as an external factor. Several thousands of copies of mitochondrial DNA are replicated at random and unequally distributed to daughter cells according to probability law. Therefore, the mitochondrial DNA is characterized in that the occurrence of mutant mitochondrial DNA falls into heteroplasmy in which the mutant coexists with normal mitochondrial DNA. Hence, even if parent cells have both normal mitochondrial DNA and mutant mitochondrial DNA, only any one of them may be present, i.e., homoplasmy may occur, in some daughter cells obtained by cell division. In this way, somatic cells having an increased copy number of unhealthy mitochondria are responsible for developing various pathological conditions in individuals.

Mitochondrial disease is diagnosed, mainly, with reference to characteristic central nervous symptoms, and brings about abnormality in the organs of the body from skeletal muscle and the heart to endocrine glands (http://www.nanbyou.orjp/entry/335). For the typical mitochondrial disease MELAS (mitochondrial encephalomyopathy, lactic acidosis, and stroke-like episodes) (Nature, 348 (6302): 651-3, 1990), A3243G mutation, a point mutation of a base at position 3243 from an adenine residue to a guanine residue, in tRNA (MT-TL1) gene of leucine encoded by mitochondrial DNA (mtDNA) has been identified as the most common mutation. This disease, called mitochondrial encephalomyopathy, lactic acidosis, and stroke-like episodes, is mitochondrial disease that affects the largest number of patients. The A3243G mutation is also known as a mutation that causes mitochondrial diabetes mellitus, and is reportedly found, particularly, in approximately 1% of approximately 7,000,000 Japanese diabetes mellitus patients (Nat genet. 1 (5): 368-71, 1992; and N Engl J Med. 330 (14): 962-8, 1994). The A3243G mutation causes a mutation in leucine of tRNA and causes deficiency in taurine modification. Further, a function of respiratory chain complex I is decreased, resulting in mitochondrial dysfunction (Am J Hum Genet. 49 (3): 590-9, 1991). For MELAS, taurine has thus been approved as a medicament, and the high-dose intake of taurine ameliorates symptoms. Since it is known that mitochondrial disease differs in the ratio between normal mtDNA and mutant mtDNA among organs and the difference in the ratio is a determinant of the severity or symptoms of the disease (Proc Natl Acad Sci USA, 88 (23): 10614-8, 1991), the development of a treatment method for decreasing the proportion of mutant mtDNA is expected for a radical treatment method.

Although debate on the relation of mitochondrial abnormality to cancer is still continuing, it has been reported that a mutation in mtDNA in a Lewis lung cancer-derived cultured cell line correlates with cancer metastasis (Science, 320 (5876): 661-4, 2008), and it has been further reported that diabetes mellitus or tumorigenesis in mice having mtDNA abnormality is suppressed by the administration of a ROS inhibitor (Proc Natl Acad Sci USA, 109 (26): 10528-33, 2012). It is considered that mtDNA having G13997A mutation derived from highly metastatic A11 cells reduces the activity of respiratory chain complex I, causes increased production of ROS and decreased production of ATP, and activates metastasis-related gene Mcl-1 or Hif-1α present in the nuclei, thereby increasing cancer metastasis (Science, 320 (5876): 661-4, 2008). It has also been reported that a pathogenic mutation in ND gene in colorectal cancer patients and lung cancer patients correlates with cancer metastasis (Sci Rep. 7 (1): 15535, 2017). The comparison of the number of mutations suspected of evoking decreased mitochondrial respiratory chain complex functions between metastatic primary tumor and nonmetastatic primary tumor has revealed that the metastatic primary tumor has a significantly high mtDNA mutation rate and more frequently exhibits heteroplasmy than the nonmetastatic primary tumor (Sci Rep. 7 (1): 15535, 2017; and Oncogene, 36 (31): 4393-4404, 2017). Cancer is also characterized in that homoplasmy is observed in many cases. This suggests that a mutation in mtDNA influences the malignancy of not only mitochondrial disease but also cancer, and cancer may be treated by targeting mutant mitochondrial DNA.

The DNA binding compound that binds to a specific sequence of DNA of a double-membrane organelle includes pyrrole-imidazole polyamide (also referred to as PIP or PI polyamide). The DNA binding compound that binds to a specific sequence of DNA of a double-membrane organelle is also referred to as a DNA minor groove binding compound.

The DNA binding compound that binds to a specific sequence of DNA of a double-membrane organelle used in the present invention is a linear DNA binding compound.

The DNA binding compound that binds to a specific sequence of DNA of a double-membrane organelle used in the present invention is also referred to as a mtDNA sequence recognizing linear PIP compound.

PIP is an oligomer comprising N-methylpyrrole (Py) and N-methylimidazole (Im) synthesized with reference to a natural antibiotic structure such as a natural compound distamycin or duocarmycin produced by bacteria, and binds to a minor groove of DNA via a hydrogen bond. The nucleotide sequence recognition depends on a combination of Py and Im. Since Py/Py recognizes A/T or T/A, Py/Im recognizes C/G, and Im/Py recognizes G/C, a dimer is formed with not a linear structure common in natural compounds but a hairpin structure which binds to DNA more strongly, and synthesized and used so as to recognize arbitrary double-stranded DNA in a nucleotide sequence-specific manner (Bioorg Med Chem. 9 (9): 2215-35, 2001). Also, it has been reported that PIP suppresses gene expression by binding to DNA in a nucleotide sequence-specific manner and inhibiting the transcription of the gene. It has also been reported that the linear polyamide distamycin is localized in mitochondria, Golgi body, and ER (Bioorganic & Medicinal Chemistry Letters 11 (2001) 769-772). It is also known that hairpin PIP is capable of being integrated into the mitochondrial genome. However, any efficient method for decreasing a mutant mitochondrial DNA copy number and increasing the number of normal mitochondrial DNA has not been developed. Neither a method that can maintain a mitochondrial function in the course thereof nor a compound that is easily synthesized and is more suitable for drug development has been conceived.

On the basis of these problems under present circumstances, the present inventors have believed that the problems are solved by synthesizing a conjugate that delivers a low-molecular-weight DNA minor groove binding compound to mitochondria and allows the compound to be retained therein, and conducted diligent studies. It is thus considered that a compound that eliminates mitochondria having a specific mtDNA sequence and increases the number of normal mitochondria, or a conjugate compound that induces cell death in cells having many copies of a specific mtDNA sequence may be developed; and the synthesis of such a conjugate is easy and its inexpensive production and delivery to organs, cells, or mitochondria may be more efficiently performed. On the basis of this novel idea, the design and synthesis of the compound of the present invention have been attempted, and a plurality of conjugate compounds thus synthesized have been confirmed to recognize mutant mtDNA, presumably causing decrease in mutant mtDNA copy number, increase in total mtDNA copy number and the induction of cell death. Thus, it has been found that the compound of the present invention is capable of serving as a drug that can produce a radical treatment method for a mitochondria-related pathological condition.

From results of Examples of the present invention, it is believed that the problems can be solved by use of a method for synthesizing a conjugate compound of a sequence-specific mtDNA sequence recognizing linear polyamide compound and a mitochondrial delivery substance such as a lipophilic cation; and a therapeutic agent that can potentially induce mitophagy in mitochondria having a specific DNA sequence, induce cell death in pathogenic cells, and radically treat a mitochondria-related pathological condition can be synthesized.

The mtDNA sequence recognizing linear polyamide serving as a component of the conjugate of the present invention is polyamide designed so as to recognize a sequence having a mtDNA polymorphism and a somatic mutant sequence. The mtDNA sequence and its polymorphism and mutant sequence are obtained from a database such as MITOMAP (A human mitochondrial genome database), GiiB-JST mtSNP (mitochondrial single nucleotide polymorphism), MitoDat (Mendelian Inheritance and the Mitochondrion), COSMIC (the Catalogue Of Somatic Mutations In Cancer), or GOBASE (A database of mitochondrial and chloroplast information), though the approach for obtainment is not limited to the database. The phrase "recognize a mtDNA sequence" means that a mtDNA nucleotide sequence recognizing polyamide binds thereto (e.g., binds through a hydrogen bond or cross-linking), for example. Examples of the mtDNA sequence recognizing compound can include the pyrrole-imidazole polyamide (PIP) described above as well as peptide nucleic acid (PNA), bridged nucleic acid, locked nucleic acid (LNA), DNA binding proteins such as zinc finger and chimeric proteins thereof, and DNA binding compounds such as guide RNA-protein conjugates. Further, a modified product having a modification so as to maintain or improve the ability to bind to DNA is also included therein. Examples of the modified product of PIP include modified products having amine by the elongation of an alkyl chain from the methyl group of N-methylpyrrole or N-methylimidazole of PIP, lysine and arginine having an amino group on a side chain, glutamic acid having a carboxyl group on a side chain, modified products in which the modified products described above are modified with a molecule such as FITC or biotin, modified products in which PIP is N-terminally modified with a molecule such as FITC or biotin, and modified products in which PIP is C-terminally modified with a molecule such as isophthalic acid. In a preferred aspect of the present invention, a component that recognizes a sequence of double-membrane organelle DNA or chloroplast DNA for plants is included.

The pyrrole-imidazole polyamide (PIP) is polyamide containing a N-methylpyrrole unit (Py), a N-methylimidazole unit (Im), and a linear amino acid derivative moiety, and Py and Im are linked to the linear amino acid through an amide bond (-C(=O)-NH-) (Trauger et al., Nature, 382, 559-61 (1996); White et al., Chem. Biol., 4, 569-78 (1997); and Dervan, Bioorg. Med. Chem., 9, 2215-35 (2001)). PIP forms a dimer in which two chains each containing Py and Im are arranged in parallel. When a pair of Py and/or Im between the two chains is a particular combination (Py/Im pair, Im/Py pair, Py/Py pair, or Im/Im pair), the pair can bind with high affinity to a particular base pair in DNA. For example, the Py/Im pair can bind to a C-G base pair, and the Im/Py pair can bind to a G-C base pair. The Py/Py pair can bind to both an A-T base pair and a T-A base pair. For example, in the case of targeting the nuclear genome or the like in order to further stabilize this dimer formation, a stronger bond is obtained by synthesizing PIP having a conformation folded as a whole via the linear amino acid derivative and such PIP is generally used (White et al., Chem. Biol., 4, 569-78 (1997); and Dervan: Bioorg. Med. Chem., 9, 2215-35 (2001)). However, for the formation of this folded conformation on hairpin, it is necessary to form a conjugate having a larger molecular weight. It has been considered that sequence recognition by a linear DNA recognizing compound may suffice for double-membrane organelle DNA having a small genome size. PIP may additionally contain 3-hydroxypyrrole (Hp) or β-alanine. As for Hp, the Hp/Py pair can bind to a T-A base pair (White et al., Nature, 391, 468-71 (1998)). PIP may be N-terminally modified not only with an acetyl group but with a molecule such as FITC or biotin. The β-alanine/β-alanine pair can bind to a T-A base pair or an A-T base pair. Accordingly, PIP that recognizes a regulatory region of a target gene can be designed by changing, for example, the combination of a pair of Py and/or Im according to the DNA sequence of the target. Methods for designing and producing PIP are known (e.g., JP Patent No. 3045706, JP Patent Publication (Kokai) No. 2001-136974 A (2001) and International Publication No. WO2013/000683).

The bridged nucleic acid or the locked nucleic acid (LNA) recognizes a sequence so as to recognize a regulatory region of a target gene, and can be synthesized as 2',4'-BNA in which the 2'-oxygen atom and 4'-carbon atom of RNA are bridged through a methylene chain, or 2',4'-ENA (ethylene-bridged nucleic acid) in which the 2'-oxygen atom and 4'-carbon atom of RNA are bridged through an ethylene chain. LNA is also available from Proligo Biochemie GmbH.

Specifically, the DNA binding compound in the conjugate of the present invention is selected from the group consisting of bridged nucleic acid, locked nucleic acid (LNA), PNA, pyrrole-imidazole polyamide (PIP), a modified product of pyrrole-imidazole polyamide (PIP), a DNA binding protein, and a DNA binding protein complex.

Various lipophilic cations and the like can be localized in double-membrane organelles and used as delivery compounds for the organelles. Specifically, the delivery substance localizable in a double-membrane organelle that can be used in the conjugate of the present invention includes a lipophilic cation. Examples of the lipophilic cation include TPP (triphenylphosphonium), alkyltriphenylphosphonium cations, rhodamine, cyanine cations, cationic peptides, guanidinium, triethylammonium, pyridinium, 3-phenylsulfonylfuroxan, F16, 2,3-dimethylbenzothiazo-lium iodide, rhodamine 19, rhodamine 123, and DQA.

Among them, TPP (triphenylphosphonium) is suitably used as the lipophilic cation. TPP is highly stable, and a conjugate comprising TPP has already been clinically applied and also confirmed to have safety (Adv Ther. 2016 Jan; 33 (1): 96-115). It has been demonstrated that TPP is incorporated at 5 to 10 times its treatment concentration into cytoplasm from the outside of the cell through plasma membrane potential difference. TPP is further capable of accumulating at 100 to 500 times its treatment concentration in mitochondrial matrix due to mitochondrial membrane potential. TPP passes directly through a lipid bilayer and is therefore distributed in mitochondria without the need of a specific incorporation system. It has also been reported that TPP delivers various substances including vitamin E, peptides, and the like to mitochondria (Proc Natl Acad Sci USA, 100 (9): 5407-12, 2003). Thus, in a preferred aspect of the present invention, TPP is a mitochondrial delivery substance having superiority in drug development according to the present invention. The structure of TPP is shown below.

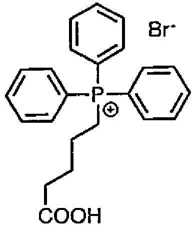

For plants, it has also been reported that the lipophilic cation TPP and its derivative SkQ1 are delivered to chloroplasts (Biochemistry (Mosc). 2015 Apr; 80 (4): 417-23. doi: 10.1134/S0006297915040045.). This suggests that the compound of the present invention interferes with mitochondria and chloroplasts which are double-membrane organelles in a DNA sequence-specific manner and can induce various bioactivities.

The delivery substance localizable in a double-membrane organelle penetrates a double-membrane organelle such as a mitochondrion and is therefore referred to as a double-membrane organelle penetration site (mitochondrion penetration site).

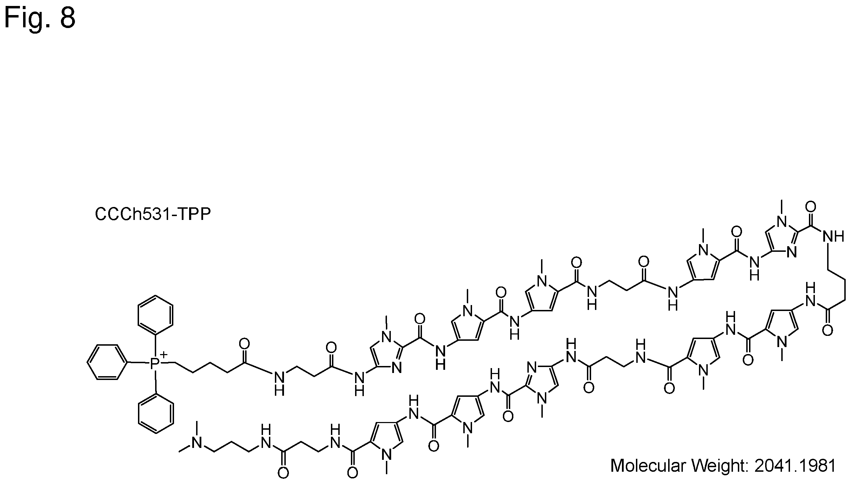

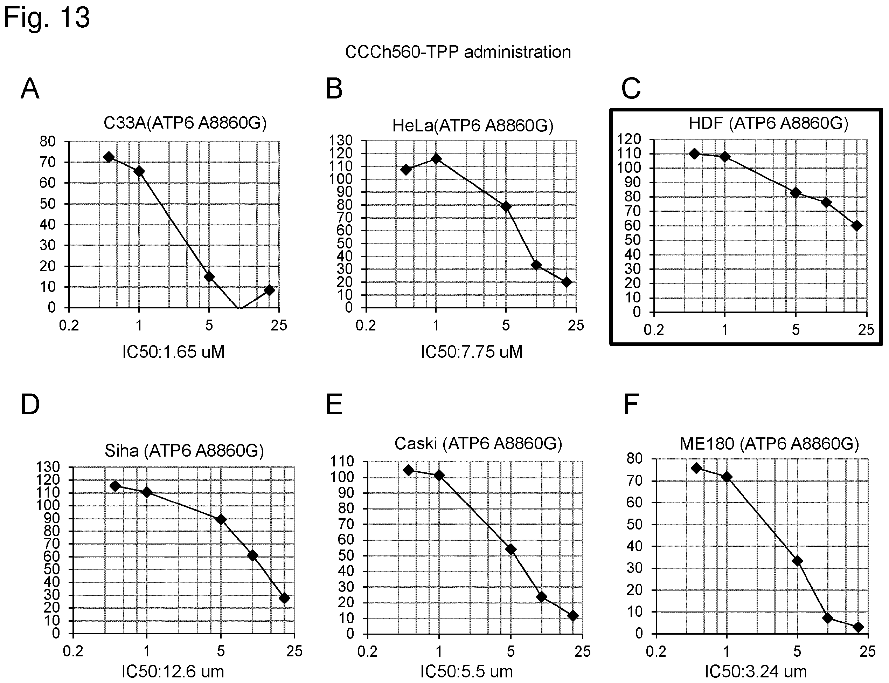

Since PIP can recognize a gene sequence, a hairpin or linear form targeting A3243G mutation of MELAS can be designed, for example, as shown in Figures 1 and 8, to synthesize a PIP compound. In order to further attempt long-term retention in mitochondria, its conjugate with the lipophilic cation TPP can be designed and synthesized. This A3243G mutation of MELAS is the most frequent mutation in mitochondrial disease. It has been reported that this mutation also participates in diabetes mellitus or cancer. As for a mtDNA polymorphism which may not be related directly to the development of a pathological condition, when mtDNA having the polymorphism coexists with a pathogenic mutation, PIP can also be designed with the sequence of the mtDNA polymorphism as a target to synthesize a conjugate compound for the A14582G polymorphism of mtDNA as shown in Figure 13. This conjugate targeting the polymorphism can eventually target the pathogenic mtDNA and may presumably be designed and synthesized as a therapeutic drug that does not influence normal mitochondria having a distinctive sequence of the same polymorphism thereas.

The conjugate of the present invention can be synthesized, for example, by attaching the polyamide to the double-membrane organelle localizable substance. The "attachment" may be performed directly or via a linker. The linker is not particularly limited as long as the linker neither impairs the effect of an alkylating agent nor the recognition of a double-membrane organelle DNA sequence. Examples thereof can include amide bonds, phosphodisulfide bonds, ester bonds, coordination bonds, and ether bonds.

The conjugate of the present invention may also contain a labeling material.

The conjugate of the DNA binding compound and the double-membrane organelle localizable substance of the present invention can modify double-membrane organelle DNA. The DNA binding compound targets a DNA sequence of mitochondria or the like, recognizes mitochondrial mutations and polymorphisms, and exhibits bioactivity such as the induction of mitophagy for the mitochondrion. Specifically, the conjugate is capable of targeting the pathological condition, such as mitochondria-related disease, described above.

The conjugate of the present invention may contain a carrier or an additive, in addition to the conjugate of the present invention, according to intended use. Examples of such a carrier and an additive include water, acetic acid, organic solvents, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants. The amount of the conjugate of the present invention used can be appropriately adjusted according to intended use.

Further examples of the conjugate of the present invention compound CCCh560-TPP with a decacyclic structure whose structure is shown in Figure 8 and formula XXIII.

The conjugate of the present invention recognizes a mitochondrial DNA mutation and polymorphism and accumulates on mitochondria in a sequence-specific manner and as such, can be used as a sequence-specific mitochondrion accumulating compound that recognizes a mitochondrial DNA mutation and polymorphism.

The conjugate of the present invention induces cell death in cells having a large mutant mitochondrial DNA copy number. On the other hand, the conjugate of the present invention induces cellular senescence for cells resistant to cell death. Combined use of the conjugate of the present invention and a senolytic drug can exert a synthetic lethal effect which potentiates the effect of the senolytic drug. Thus, the present invention encompasses a combination drug of the conjugate of the present invention and a senolytic drug and includes, for example, a combination kit of the conjugate of the present invention and a cytocidal drug. The present invention further encompasses the conjugate of the present invention for combined use with a senolytic drug.

Examples of the senolytic drug include anti-apoptotic BCL family inhibitors and MDM2 inhibitors and include Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5-dichloro-3-(4-methyl-1-piperazinyl)-6-[2-(1-piperidinyl)-1,3-thiazol-5-yl]benzo-1,4-quinone, Nutlin-3, MI-63, UBX0101, and UBX1967, and their derivatives, the structures and chemical formulas of which are shown in Figures 55-1 to 55-11. In addition, a senolytic drug described in any of U.S. Patent No. 10550378, U.S. Patent No. 10517866, U.S. Patent No. 10519197, U.S. Patent No. 10478432, U.S. Patent No. 10426788, U.S. Patent No. 1041354, U.S. Patent No. 10378002, U.S. Patent No. 1032807, and U.S. Patent No. 10195213 can be used.

The pharmaceutical composition of the present invention is a composition comprising the conjugate. The composition binds to a mitochondrial disease-related mtDNA sequence. Various diseases can be treated and prevented by administering the pharmaceutical composition into organisms. The pharmaceutical composition of the present invention can target diseases in every organism including plants which exploit double-membrane organelle double-stranded DNA for the body's defense, particularly, mammals (e.g., humans, rats, rabbits, sheep, pigs, cattle, cats, dogs, and monkeys). Examples of the disease targeted by the pharmaceutical composition of the present invention include diseases associated with a mutation in mtDNA, for example, mitochondrial disease, cancer, cardiocirculatory disease, cranial nervous disease/psychiatric disease, myopathy, kidney disease, liver disease, lifestyle-related disease, sleep disorder, diseases and infections with strong local symptoms in the dermatological, ophthalmological, or otological domain, allergic disease, diseases involving cellular senescence, aging, and gastrointestinal disease. Examples of the mitochondrial disease among the target diseases can include chronic progressive external ophthalmoplegia, myoclonic epilepsy and ragged-red fiber disease, mitochondrial encephalomyopathy, lactic acidosis, and stroke-like episodes (MELAS), and Leigh syndrome. Examples of the cancer can include skin cancer (malignant melanoma, basal cell skin cancer, etc.), brain tumor, head and neck cancer, esophageal cancer, tongue cancer, lung cancer, breast cancer, pancreatic cancer, stomach cancer, cancer of the small intestine or the duodenum, large intestinal cancer (colon cancer and rectal cancer), urinary bladder cancer, kidney cancer, liver cancer, prostate cancer, uterus cancer, ovary cancer, thyroid gland cancer, gallbladder cancer, throat cancer, sarcoma (e.g., osteosarcoma, chondrosarcoma, Kaposi sarcoma, myosarcoma, angiosarcoma, and fibrosarcoma), leukemia (e.g., chronic myelogenous leukemia (CML), acute myelogenous leukemia (AML), chronic lymphocytic leukemia (CLL) and acute lymphocytic leukemia (ALL), lymphoma, and multiple myeloma (MM)), pediatric solid tumor (brain tumor, neuroblastoma, hepatoblastoma, nephroblastoma, Ewing's sarcoma, etc.), retinoblastoma and melanoma. Examples of the lifestyle-related disease can include, but are not particularly limited to, hypertension and diabetes mellitus. Examples of the disease and the infection with strong local symptoms in the dermatological, ophthalmological, or otological domain can include hearing loss, psoriasis, chronic dermatitis, paranasal sinusitis, glaucoma, and retinal degeneration. Examples of the allergic disease can include atopic dermatitis and pollinosis. Examples of the disease involving cellular senescence can include skin wrinkles, sags, and pigmentation. Examples of the cranial nervous disease/psychiatric disease can include cramp, myoclonus, deconditioning, stroke-like symptoms, decreased intelligence, migraine, psychological symptoms, dystonia, myelopathy, bipolar disorder, Parkinson's disease, and dementia. Examples of the myopathy can include loss in muscle strength, easy fatiguability, and hyperCKemia. Examples of the cardiocirculatory disease can include conduction system disease, Wolff-Parkinson-White (WPW) syndrome, cardiomyopathy, and pulmonary hypertension. Examples of the kidney disease can include Fanconi syndrome, renal tubular dysfunction, glomerular lesions, and urine myoglobin. Examples of the liver disease can include hepatic function disorder and hepatic failure.

The disease targeted by the pharmaceutical composition of the present invention preferably includes every pathological condition and/or disease involving double-membrane organelle double-stranded DNA. The target disease also includes every disease derived from mitochondria-related disease. The pharmaceutical composition of the present invention acts more effectively on cells having a large copy number of mitochondrial DNA having a target sequence, promotes mitophagy, and induces decrease in the number of mitochondria having the target sequence, or a cell death mechanism ascribable to mitochondrial dysfunction for particular cells, thereby normalizing or killing the disease causative cells. Thus, the disease can be treated and prevented.

The pharmaceutical composition of the present invention can be in any dosage form of oral administration and parenteral administration. These dosage forms can be formulated according to a routine method and may contain a pharmaceutically acceptable carrier or additive. Examples of such a carrier and an additive include water, acetic acid, pharmaceutically acceptable organic solvents, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants acceptable as pharmaceutical additives.

The additive is selected alone or in appropriate combination from among those described above according to the dosage form of the pharmaceutical composition of the present invention. The dosage form for oral administration is tablets, capsules, fine granules, powders, granules, solutions, syrups, external preparations, or the like, and the pharmaceutical composition of the present invention may be administered through aerosolized agents, embrocations, eye drops, or appropriate dosage forms. Examples of the dosage form for parenteral administration include injections. The injection can be administered systemically or locally through intravenous injection such as drip infusion, subcutaneous injection, intraperitoneal injection, intratumoral injection, or the like.

For example, for use as an injectable preparation, the pharmaceutical composition of the present invention is dissolved in a solvent (e.g., saline, a buffer solution, a glucose solution, 0.1% acetic acid, and polyoxyethylene hydrogenated castor oil). This solution can be supplemented with an appropriate additive (human serum albumin, PEG, mannose-modified dendrimer, cyclodextrin conjugate, etc.) for use. Alternatively, the pharmaceutical composition of the present invention may be freeze-dried for a dosage form that is dissolved before use. For example, sugar alcohols or saccharides such as mannitol and glucose, and polymerized polysaccharides such as dextran can be used as excipients for freeze drying.

For example, for use as a transdermal absorption preparation, the pharmaceutical composition of the present invention is dissolved in a solvent (e.g., white Vaseline, liquid paraffin, isopropyl myristate, beeswax, lanoline, stearic acid, stearyl alcohol, cetanol, glycerin, propylene glycol, 1,3-butylene glycol, ethanol, and isopropanol). This solution can be supplemented with an appropriate additive (glycerin monostearate, sorbitan monostearate, polyoxyethylene hydrogenated castor oil 60, polysorbate 60, methyl p-hydroxybenzoate, propyl p-hydroxybenzoate, phenoxyethanol, thymol, sodium bisulfite, ascorbic acid, tocopherol, dibutylhydroxytoluene, sodium edetate hydrate, benzotriazole, citric acid hydrate, sodium citrate hydrate, lactic acid, diisopropanolamine, acetic acid, sodium acetate hydrate, laurocapram, pyrrothiodecane, etc.) for use.

The dose of the pharmaceutical composition of the present invention or the compound of the present invention differs depending on age, sex, symptoms, an administration route, the number of doses, and the dosage form. The dose, for example, in a human adult (60 kg), is 0.01 to 1000 mg, preferably 0.1 to 100 mg, more preferably 1 to 30 mg, per day. The administration method is appropriately selected according to the age and symptoms of a patient. The administration may be performed once at intervals of several days and in one portion or two to four divided portions per day, for example.

The pharmaceutical composition of the present invention can be used as an anticancer agent. Examples of the type of the targeted cancer include, but are not limited to, large intestinal cancer, pancreatic cancer, colorectal cancer, thyroid gland cancer, lung cancer, neck cancer, endometrial cancer, myelodysplastic syndrome, adenocarcinoma of the thyroid gland and the colon, and neuroblastoma. The pharmaceutical composition of the present invention can target cells constituting cancer and a peritumoral microenvironment thereof, the cancer having a specific mtDNA sequence different from that of normal cells, or the cancer having an increased copy number of mtDNA dominantly having a polymorphism sequence, albeit present in normal cells. The anticancer agent of the present invention may contain a carrier or a composition according to intended use, as in the pharmaceutical composition mentioned above.

The present invention also encompasses a kit. The kit comprises the compound of the present invention as well as a pharmaceutically acceptable carrier or additive, reagents, an aid, a dedicated container, other necessary accessories, and an instruction. The kit of the present invention can also be used as a kit for cancer treatment, a kit for cell treatment, a kit for double-membrane organelle introduction treatment, a kit for diagnosis, or a research reagent kit.

### Examples

Hereinafter, the present invention will be described further specifically with reference to Examples. However, the present invention is not limited by these Examples.

### [Example 1]

Double-membrane organelle double-stranded DNA is present in organelles, for example, mitochondria or chloroplasts, present in eukaryotes including humans, animals and plants, and is deeply involved in the energy production of these organisms, such as respiration or photosynthesis, and also involved in the production of substances having biological and pathological effects, such as reactive oxygen species. This DNA is present in cytoplasm and outside a nuclear membrane and is therefore susceptible to damage, as compared with nuclear DNA, and has a property of facilitating new mutations accumulating. Such mutations accumulate with aging, falling into a state called "heteroplasmy" in which mutant mtDNA coexists with normal mtDNA in somatic cells of human adults. A given proportion or more of mutant mtDNA decreases mitochondrial functions and is responsible for the development of mitochondrial disease or the like. It is also known that mutations in mtDNA are responsible for not only mitochondrial disease but an acquired disease group of so-called lifestyle-related disease such as diabetes mellitus or cancer. In this Example, a conjugate of hairpin PIP-TPP of the formula (VII) given below (referred to as CCC018-TPP) was studied for its bioactivity against cells having the target mtDNA sequence. A sustained mitochondrial localization confirmation experiment, a mitochondrial DNA copy number analysis experiment, an expression analysis experiment of related genes, a mitochondrial autophagy reaction confirmation experiment, a cell death induction experiment of cells harboring a mutant mitochondrion, a programmed cell death confirmation experiment, a synthetic lethality induction experiment, a skin penetrability confirmation experiment, and a plant live cell penetrability confirmation experiment were conducted.

In order to study change in mitochondrial mutant DNA copy number, ρ0 cells deficient in mtDNA were first prepared from human uterine cervical cancer-derived cell line HeLa cells. The cells were fused with platelet containing mtDNA with A3243G mutation derived from a MELAS patient so that mutant mitochondrial DNA was introduced at a different proportion to prepare 3243G Low cells (HeLamt3243 cells containing 55% mutant mtDNA) and 3243G High cells (HeLamt3243 cells containing 82% mutant mtDNA). Also, the cells were fused with non-mutant mitochondrial DNA of wild-line HeLa cells to prepare HeEB 1 (HeLamtHeLa) cells. The cells were cultured at 37°C under 5% CO₂ using Dulbecco's Modified Eagle's Medium (DMEM D5796: Sigma-Aldrich, USA) containing 10% fetal bovine serum (FBS: gibco), 1% penicillin-streptomycin (gibco), 0.1 mg/ml sodium pyruvate (Sigma-Aldrich, USA), and 50 mg/ml uridine (Sigma-Aldrich).

The compound CCC018 is hairpin PIP targeting the A3243G mutant sequence GGGCCCT of mitochondrial DNA, which is a causative mutation of the mitochondrial disease MELAS or mitochondrial diabetes mellitus, and was synthesized in line with the description of International Publication No. WO2012/133896. For CCC019, the possibility of binding to mitochondrial DNA and inducing decrease in the copy number of a mutant mitochondrion was predicted. However, although the experiment described in International Publication No. WO2012/133896 was conducted, no reproducibility was obtained.

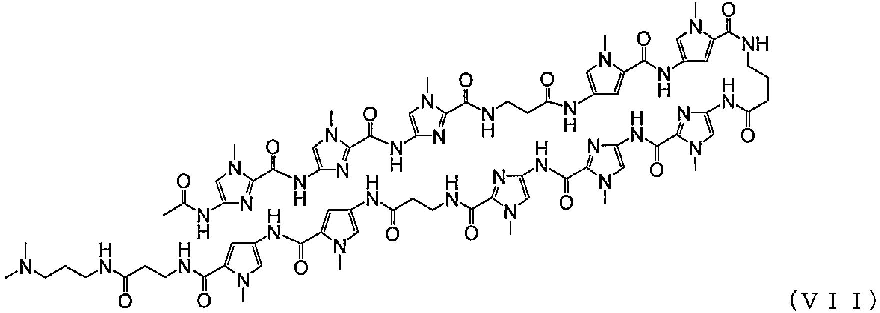

From these results and as described in Bioorganic & Medicinal Chemistry Letters 11 (2001) 769-772, DNA minor groove binding compounds were presumed to be localized in mitochondria and bind to mtDNA. However, it was considered that these compounds were translocated to Golgi body and smooth-surfaced endoplasmic reticulum and thereby translocated to an excretion system to the outside of cells by the formation of secretory vesicles, secretory granules, lysosome and endosome, etc., without continuously maintaining their effective binding states with mtDNA.

In order to confirm this, CCC018 was fluorescently labeled to synthesize CCC018-FITC (Figure 1-1), which was fused with platelet containing mtDNA with A3243G mutation derived from a MELAS patient so that mutant mtDNA was introduced at a different proportion. Under culture of the resulting 3243G High cells (HeLamt3243 cells containing 82% mutant mtDNA), the localization of red fluorescence for mitochondria and green fluorescence for CCC018-FITC was experimentally observed with Mito Tracker.

The 3243G High cells were inoculated at 1 × 10⁵ cells/dish to a 35 mm glass base dish (IWAKI, Japan), cultured for 24 hours, and then treated with CCC018-FITC (final concentration: 1 µM). Further, 24 and 48 hours later, the culture solution was discarded, and a culture solution containing Mito Tracker(R) Red CMXRos (Life Technologies, USA) was added to attain 50 nM. 10 minutes later, the culture solution was replaced with a fresh one, followed by observation under confocal laser microscope SP8 (Leica, Germany).

As shown in Figure 1-2, fluorescent images were confirmed in which the colocalization of green fluorescence with red fluorescence of some mitochondria was found 24 hours after CCC018-FITC administration, whereas the colocalization of green fluorescence with mitochondria was not found 48 hours thereafter and the compound was presumably translocated to Golgi body or smooth-surfaced endoplasmic reticulum, as in the literature (Bioorganic & Medicinal Chemistry Letters 11 (2001) 769-772). This suggested that hairpin PIP is localized in mitochondria in a limited manner and cannot efficiently decrease the number of mutant mitochondria.

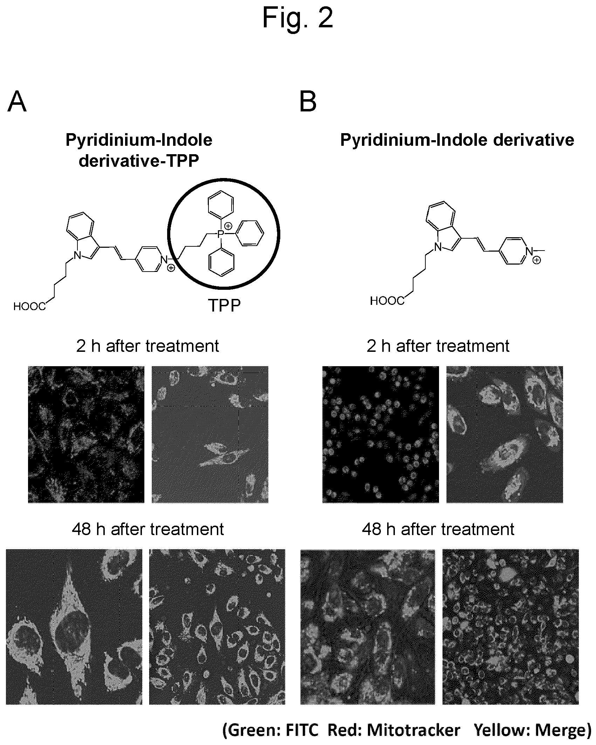

In order to solve the problems, it was believed that mitochondrial localization was able to be improved by condensing a lipophilic cation whose mitochondrial localization had been reported with hairpin PIP. (4-Carboxybutyl)triphenylphosphonium bromide (TPP, Sigma-Aldrich) was purchased, and mitochondrial localization was confirmed. The intracellular localization of pyridinium-indole derivative-TPP in which TPP was attached to a pyridinium group with indole as a linker (formula (VIII) given below) and a comparative pyridinium-indole derivative without the addition of TPP (formula (IX) given below) was observed 2 hours and 48 hours after treatment as described above. As shown in Figure 2, the pyridinium-indole derivative-TPP was colocalized with mitochondria, and this colocalization was found even 48 hours after treatment, whereas the colocalization of the pyridinium-indole derivative without the addition of TPP with mitochondria was not found.

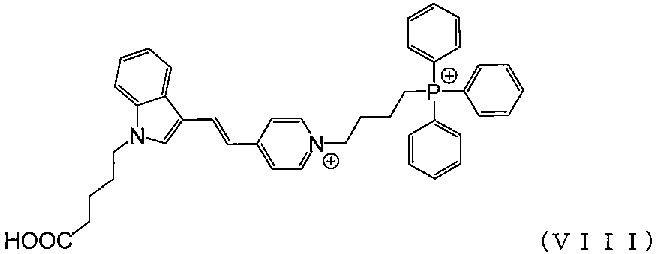

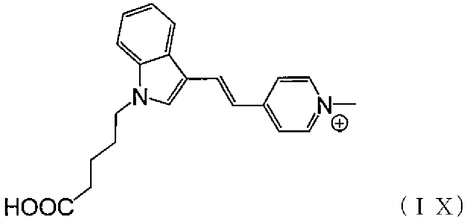

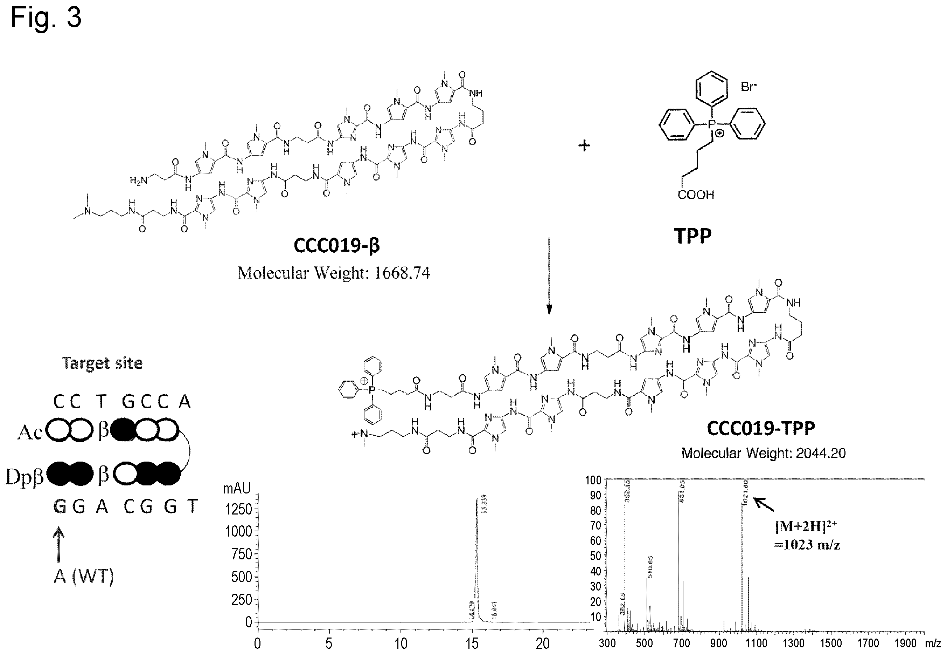

On the basis of the description above, CCC019-TPP (formula (X) given below) was synthesized, as shown in Figure 3, as a PIP-TPP compound in which TPP was condensed with hairpin PIP CCC019 targeting the A3243G mutant sequence CCTGCCA of mtDNA. CCC019 is a compound that recognizes the A3243G mutant sequence, as in CCC018, but exhibits more improved synthesis efficiency through fewer consecutive bonds of imidazole groups.

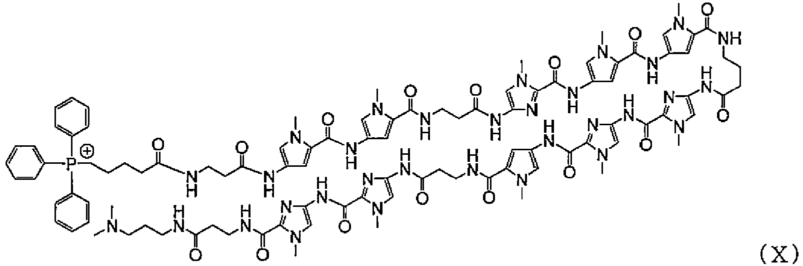

The intracellular localization of CCC018-TPP was studied as described below. The mtDNA mutation cell line 3243G Low (HeLamt3243 cells containing 55% mutant mitochondrial DNA) or 3243G High cells were inoculated at 2 × 10⁴ cells/dish to a 35 mm glass base dish (IWAKI), cultured for 24 hours, and then treated with CCC018-TPP (final concentration: 500 nM for the 3243G Low cells and 100 nM for the 3243G High cells). The 3243G Low cells were reseeded at 2 × 10⁴ cells/dish before becoming confluent, and treated for 14 days after replacement with a fresh culture solution containing CCC018-TPP. The 3243G High cells were treated for 3 days without replacement of the culture solution. After the completion of CCC018-TPP treatment, the cells were fixed with 4% paraformaldehyde (PFA)/PBS at room temperature for 30 minutes. The cells thus fixed were washed with PBS and blocked with 10% FBS/0.1% Triton X100/TBS (TBST) for 10 minutes. TBS is a mixed solution of sodium chloride (final concentration: 150 µM) and Tris (pH 7.4) (final concentration: 20 µM). After the completion of blocking, the cells were reacted overnight at 4°C with primary antibodies diluted with TBST. The primary antibodies used were a rabbit anti-TPP antibody (kindly provided by Dr. M. Murphy from the University of Cambridge, UK) diluted at 1:500, and a mouse anti-cytochrome C antibody (Thermo Fisher Scientific, USA) diluted at 1:500. After the completion of reaction with the primary antibodies, the cells were washed three times with TBS and reacted for 15 minutes with secondary antibodies diluted with TBST, i.e., Goat anti Rabbit Oregon Green 488 (Invitrogen, USA) diluted at 1:1000 and Goat anti Mouse Alexa Fluor 647 (Invitrogen) diluted at 1:1000. After the completion of reaction with the secondary antibodies, the cells were washed three times with TBS, mounted in DABCO/PVA mounting medium (Sigma-Aldrich), and observed under confocal laser microscope SP8 (Leica).

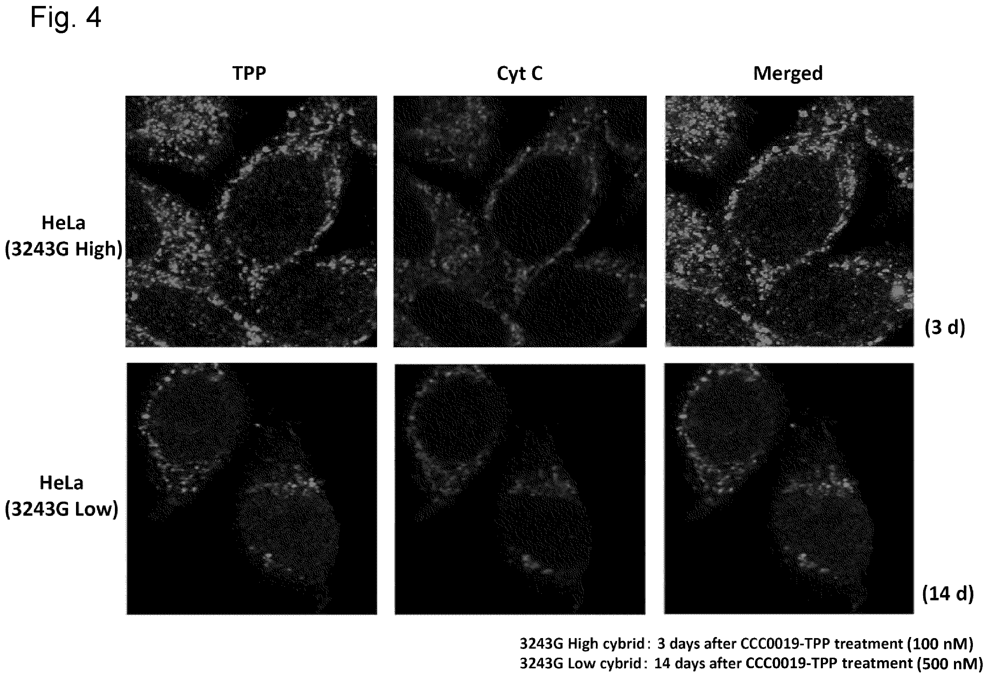

As a result of treating two types of cells having the 3243G mutation with CCC018-TPP, findings about its colocalization with CytC present in mitochondrial inner membranes were observed in the 3243G High cells 3 days after CCC018-TPP administration, and even 14 days after CCC018-TPP administration to the 3243G Low cells having a small number of mutant mitochondria (Figure 4). Thus, CCC018-TPP was confirmed to be delivered to mitochondria and retained therein for a long period.

In order to analyze change in total mitochondrial DNA copy number after CCC019-TPP treatment, a quantitative experiment was conducted by PCR as described below.

The 3243G Low cells were inoculated at 2 × 10⁴ cells/well to a 35 mm dish (Falcon, USA), cultured for 24 hours, and then treated with CCC019-TPP (final concentration: 1 µM and 5 µM). The medium was replaced once two days, and CCC019-TPP was continuously administered. Further, 48 and 63 days later, DNA was extracted using Allprep DNA/RNA Mini Kit (QIAGEN, Germany) according to the instruction manual. The yield of the DNA thus extracted was measured using NANODROP 2000c (Thermo Fisher Scientific). DNA samples were prepared, and PCR reaction and analysis were conducted. The primers and reaction conditions used are shown below.
- COII
   Forward primer: ACA CAT TCG AAG AAC CCG TAT (SEQ ID NO: 1)
   Reverse primer: TTT AGT TGG GGC ATT TCA CTG (SEQ ID NO: 2)
- β actin (internal control)
   Forward primer: TGA CGG GGT CAC CCA CAC TGT GCC CAT CTA (SEQ ID NO: 3)
   Reverse primer: CTA GAA GCA TTT GCG GTG GAC GAT GGA GGG (SEQ ID NO: 4)

The reaction conditions were as follows.
• COII (20 cycles)

| | |
|---|---|
| Initial denaturation | 94°C for 1 min |
| Denaturation | 94°C for 30 sec |
| Annealing | 53°C for 30 sec |
| Extension | 72°C for 1 min |
| Final extension | 72°C for 1 min |

• β actin (25 cycles)

| | |
|---|---|
| Initial denaturation | 96°C for 5 min |
| Denaturation | 96°C for 30 sec |
| Annealing | 59°C for 30 sec |
| Extension | 72°C for 30 sec |
| Final extension | 72°C for 5 min |

After the completion of PCR reaction, PCR products were electrophoresed on 2% agarose gel and then stained with ethidium bromide to detect bands of the PCR products. The bands were quantified with analytical software (Image J).

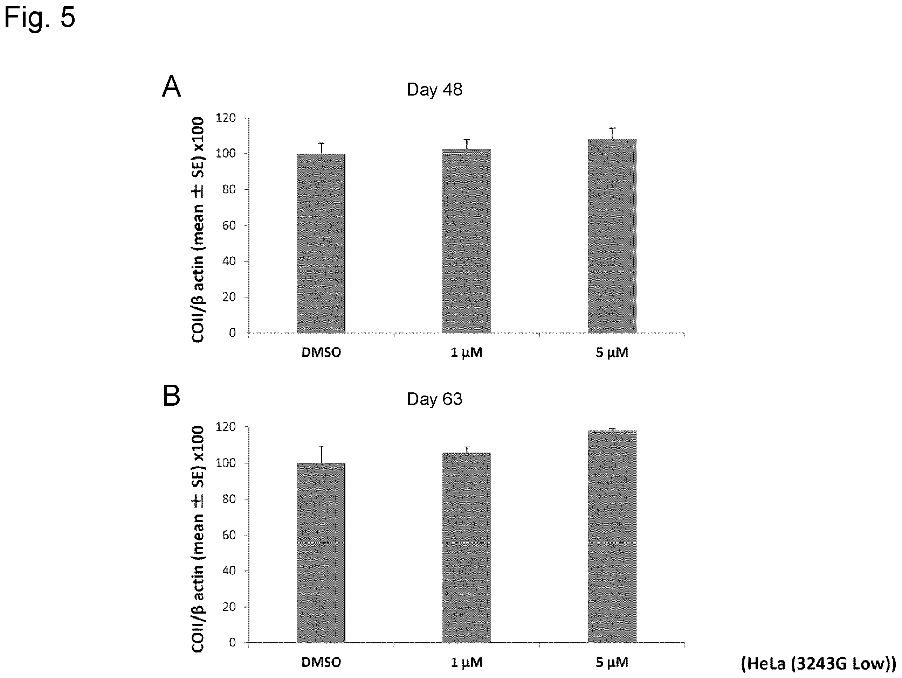

In order to predict the total copy number of mtDNA in the 3243G Low cells treated with CCC019-TPP for 48 or 63 days, COII gene constituting mitochondrial respiratory chain complex IV was subjected to PCR. As a result, the tendency to increase the total copy number of mtDNA in a CCC019-TPP concentration-dependent manner was observed in both the experiments involving the 48- or 63-day treatment (Figure 5).

In order to analyze change in the ratio between normal mtDNA and mutant mtDNA by CCC019-TPP treatment, analysis was conducted by PCR-RFLP as described below.

The 3243G Low cells were inoculated at 2 × 10⁴ cells/well to a 35 mm dish (Falcon), cultured for 24 hours, and then treated with CCC019-TPP (final concentration: 1 µM and 5 µM). The medium was replaced once two days, and CCC019-TPP was continuously administered. Further, 48 and 63 days later, DNA was extracted using Allprep DNA/RNA Mini Kit (QIAGEN) according to the instruction manual. The yield of the DNA thus extracted was measured using NANODROP 2000(TM) (Thermo Fisher Scientific). DNA samples were prepared, and PCR reaction was conducted. The primers and reaction conditions used are shown below.
- mt3243
   Forward primer: TTC ACA AAG CGC CTT CCC CCG T (SEQ ID NO: 5)
   Reverse primer: GCG ATG GTG AGA GCT AAG GTC GG (SEQ ID NO: 6)

The reaction conditions were as follows.

### (25 cycles)

| | |
|---|---|
| Initial denaturation | 95°C for 5 min |
| Denaturation | 95°C for 30 sec |
| Annealing | 57°C for 30 sec |
| Extension | 72°C for 30 sec |
| Final extension | 72°C for 5 min |

PCR products were treated with restriction enzyme ApaI as described below, and analyzed. The PCR products were purified using QIAquick PCR Purification Kit (QIAGEN) according to the instruction manual. After the purification, the yield of DNA was measured using NANODROP 2000c (Thermo Fisher Scientific). After the measurement, reaction products amplified in PCR System 9700 (Applied Biosystems, USA) GeneAmp(R) were treated with restriction enzyme ApaI at 37°C for 5 hours. After the completion of reaction, 15 ng of samples was electrophoresed on 2% agarose gel and then stained with ethidium bromide to detect bands of the PCR products. The bands were quantified with analytical software (Image J).

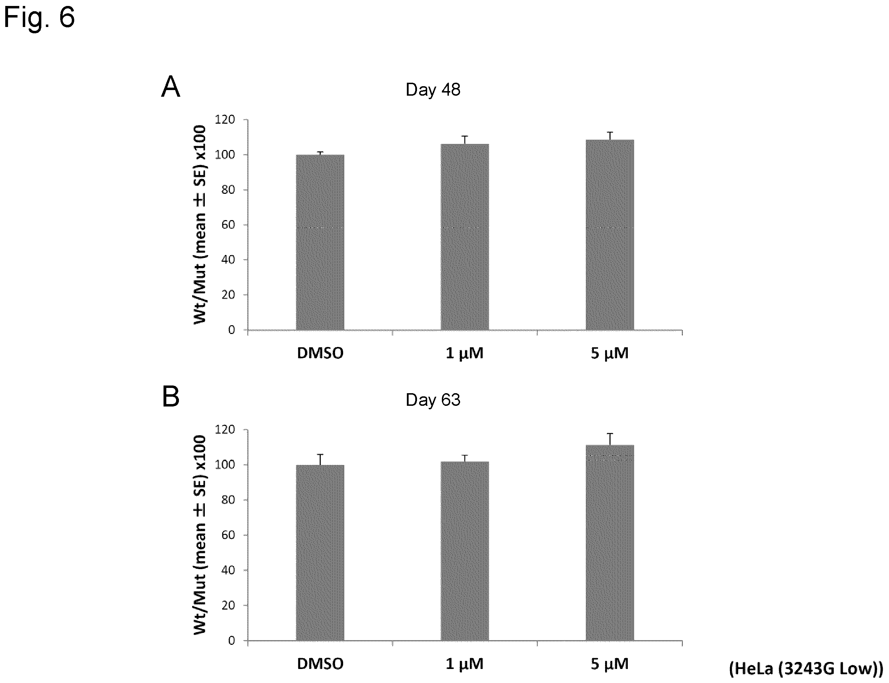

In order to examine change in the ratio between normal mtDNA and mutant mtDNA, PCR-RFLP was performed using DNA extracted from the 3243G Low cells treated with CCC019-TPP for 48 or 63 days. As a result, the tendency to increase the proportion of wild-type mitochondrial DNA and decrease the proportion of mutant mitochondrial DNA in a CCC019-TPP concentration-dependent manner was observed in both the experiments involving the 48- or 63-day treatment (Figure 6).

In order to study whether the decrease in the number of mutant mtDNA by PIP-TPP was caused by mitochondrial autophagy reaction, the following experiment was conducted. The mtDNA mutation cell line 3243G High cells were inoculated at 2 × 10⁴ cells/dish to a 35 mm glass base dish (IWAKI), cultured for 24 hours, and then treated with the compound (final concentration: 100 nM). 52 hours after treatment, the cells were fixed with 4% paraformaldehyde (PFA)/PBS at room temperature for 30 minutes. The cells thus fixed were washed with PBS and blocked with 10% FBS/0.1% Triton X100/TBS (TBST) for 10 minutes. TBS is a mixed solution of sodium chloride (final concentration: 150 µM) and Tris (pH 7.4) (final concentration: 20 µM). After the completion of blocking, the cells were reacted overnight at 4°C with primary antibodies diluted with TBST. The primary antibodies used were a rabbit anti-LC3 antibody (Cell Signaling Technology, Inc.) diluted at 1:200, and a mouse anti-cytochrome C antibody (Thermo Fisher Scientific, USA) diluted at 1:500. After the completion of reaction with the primary antibodies, the cells were washed three times with TBS and reacted for 15 minutes with secondary antibodies diluted with TBST, i.e., Goat anti Rabbit Oregon Green 488 (Invitrogen) diluted at 1:1000 and Goat anti Mouse Alexa Fluor 647 (Invitrogen) diluted at 1:1000. After the completion of reaction with the secondary antibodies, the cells were washed three times with TBS, mounted in DABCO/PVA mounting medium (Sigma-Aldrich), and observed under confocal laser microscope SP8 (Leica).

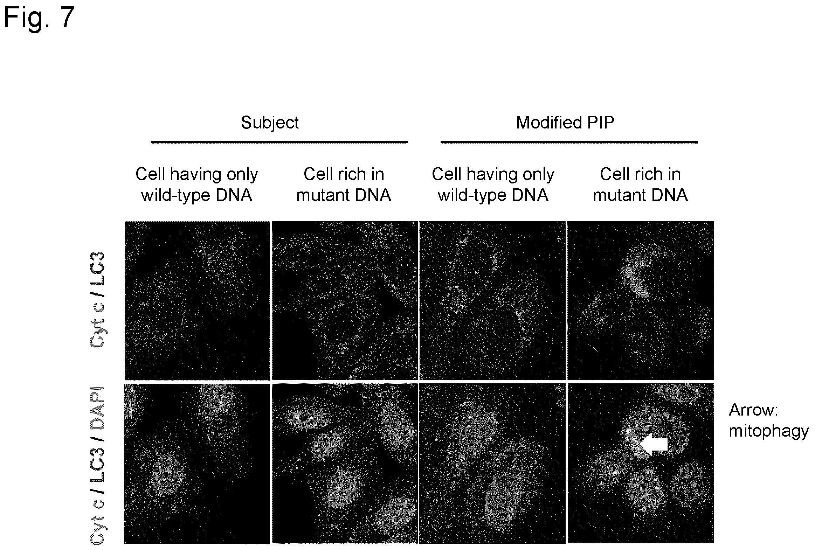

As shown in Figure 7, as CytC allows mitochondria to exhibit red fluorescence and the autophagic effect marker LC3 to exhibit green fluorescence, any finding about colocalization with the fluorescence of mitochondria was not found for the treatment with PIP, though the autophagic effect was sporadically seen in the cytoplasm. By contrast, yellow to orange fluorescence ascribable to the colocalization of red and green fluorescence was strongly found in the cytoplasm of the cells treated with PIP-TPP, and the colocalization of the autophagic effect with mitochondria was clearly observed. This suggests that treatment with PIP-TPP induced mitochondrial autophagy, i.e., mitophagy.

### [Example 2] Suppression of tumor cell proliferation by library compound

Figure 8 shows the structure of the compound CCCh531-TPP with a decacyclic structure.

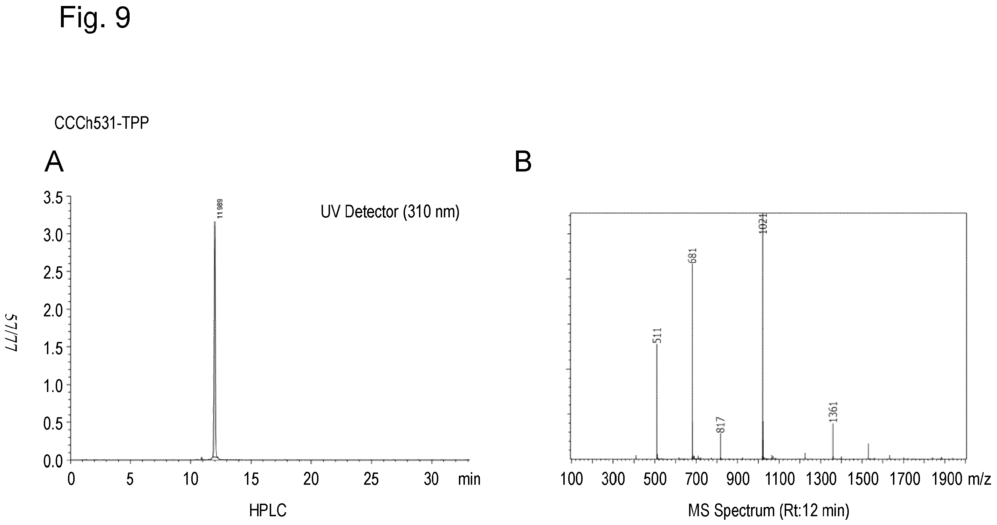

Figure 9 shows results of high-performance liquid chromatography analysis and mass spectrometry of CCCh531-TPP.

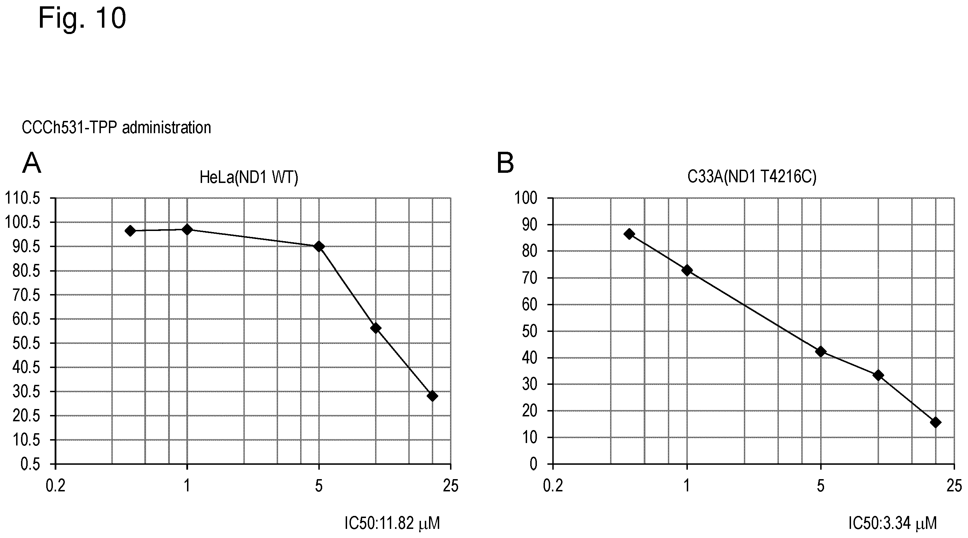

As shown in Figure 10, CCCh531-TPP (hairpin) was found to strongly suppress the proliferation of C33A with IC50 = 3.34 µM against IC50 = 11.82 µM for the HeLa cells.

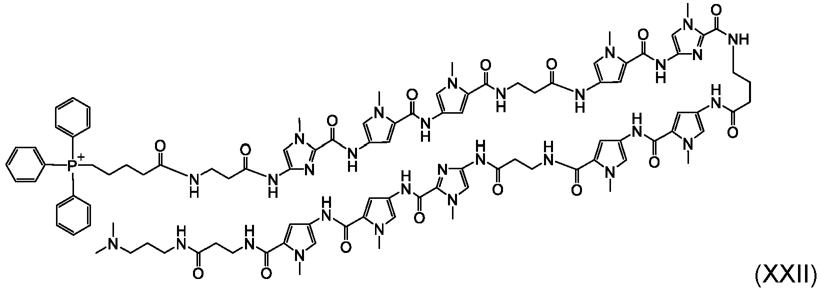

Compound CCCh560-TPP with a decacyclic structure targeting the A8860G polymorphism of mitochondrial gene ATP6 found in human populations, not acquired mitochondrial mutations in somatic cells, was studied for whether the suppression of cell proliferation was also found by 5-day treatment with the compound for the mitochondrial DNA polymorphism in cancer cell lines C33A, HeLa, Siha, Caski, and ME180 having this polymorphism in homoplasmy and human nontumor skin-derived fibroblast HDF cells. For this purpose, cell proliferation assay was conducted in the same manner as in Example 1. 1000 cells were inoculated to a 96-well plate, and CCCh560-TPP was administered at 0.5, 1, 5, 10, and 20 µM. 5 days later, WST assay was conducted.

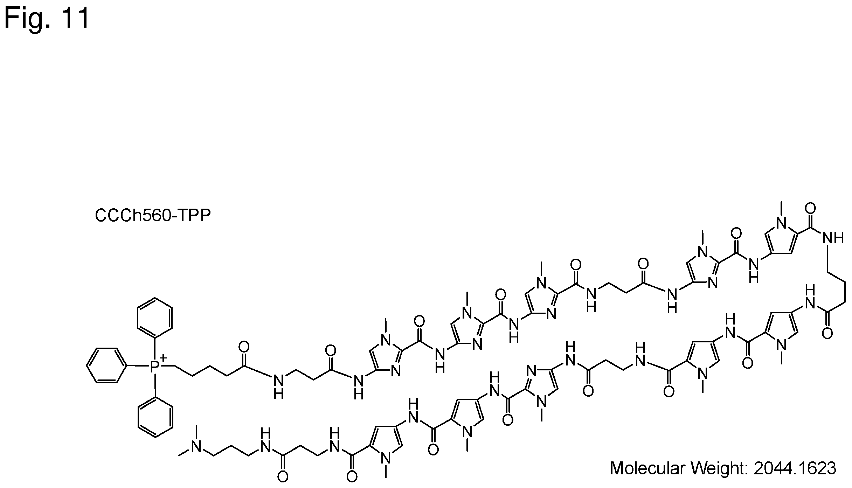

Figure 11 shows the structure of the compound CCCh560-TPP with a decacyclic structure.

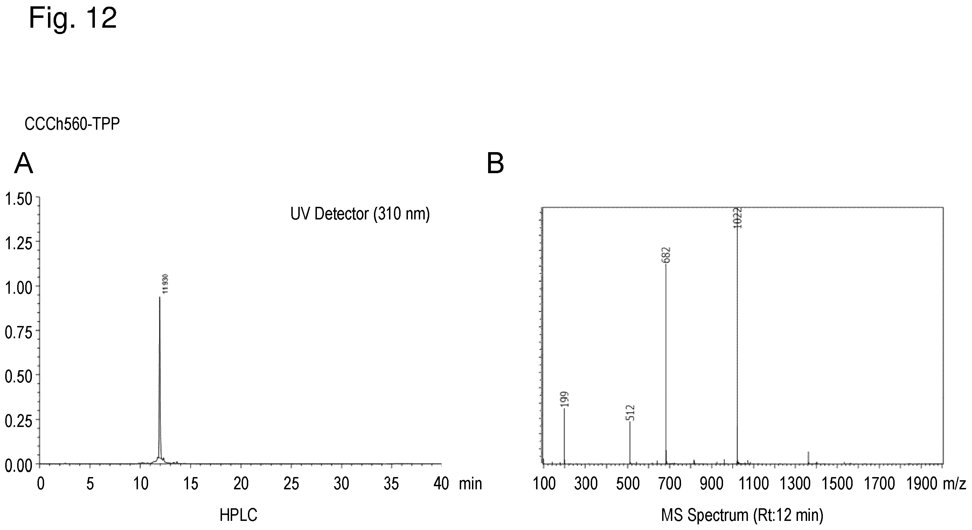

Figure 12 shows results of high-performance liquid chromatography analysis and mass spectrometry of CCCh560-TPP.

As shown in Figure 13, CCCh560-TPP exhibited the tendency to suppress the proliferation of the human nontumor cells HDF (human skin fibroblasts), albeit with IC50 incalculable. However, significantly strong suppression of proliferation was found in the tumor cells with IC50 = 1.65, 7.75, 12.6, 5.5, and 3.24 µM for the tumor cell lines C33A, Hela, Siha, Caski, and ME180, respectively.

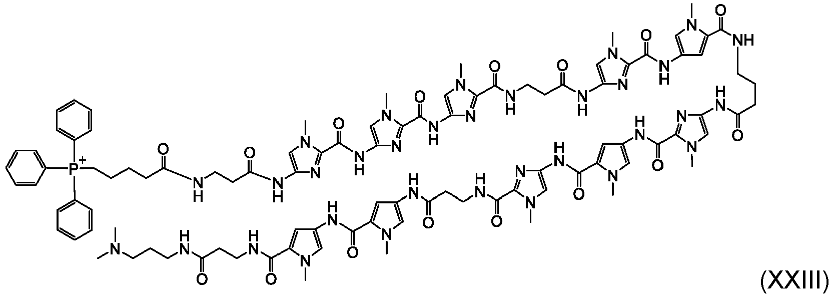

### Industrial Applicability

The conjugate of the present invention recognizes a sequence of double-membrane organelle DNA, promotes mitochondrial autophagy reaction, induces decrease in the copy number of a mutant mitochondrion and the cell death of cells having mutant mitochondria, and can be used as an active ingredient in a pharmaceutical composition for mitochondria-related disease or the like.

### Free Text of Sequence Listing

SEQ ID NOs: 1 to 30 - Primer

## Claims

1. A conjugate of a DNA binding compound that specifically binds to a sequence of double-membrane organelle DNA, and a double-membrane organelle localizable compound, wherein the conjugate has a structure represented by the following formula (X), (XXII) or (XXIII): 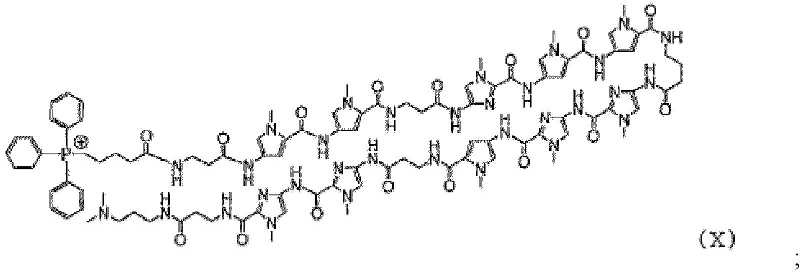 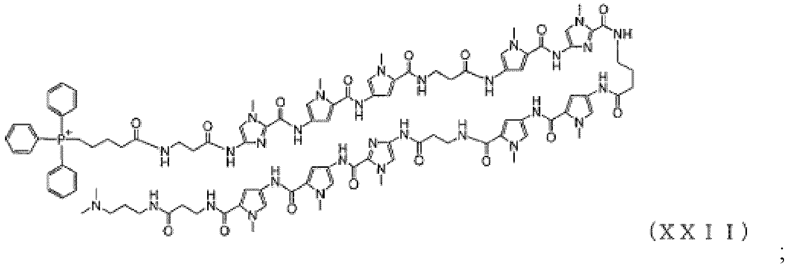 or

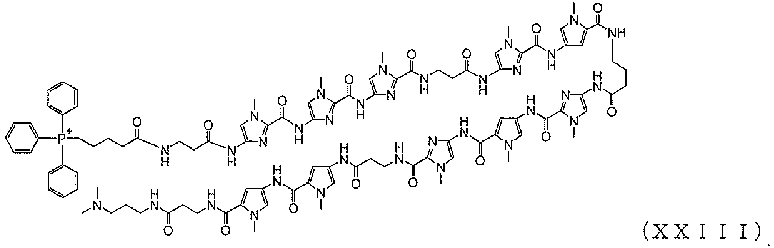

2. A conjugate of a DNA binding compound that specifically binds to a sequence of double-membrane organelle DNA, and a double-membrane organelle localizable compound, wherein the DNA binding compound has a structure represented by the following formula (VII) and the double-membrane organelle localized compound is TPP (triphenylphosphonium), wherein the compound of formula (VII) and the TPP are attached directly or *via* a linker:

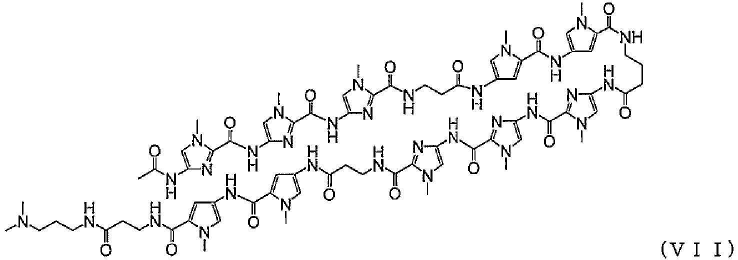

3. A composition that binds to a mitochondrial disease-related mitochondrial DNA sequence, comprising a conjugate according to either claim 1 or claim 2.

4. A pharmaceutical composition comprising a conjugate according to either claim 1 or claim 2.

5. The conjugate according to either claim 1 or claim 2, or the pharmaceutical composition according to claim 5, which is further combined with a cytocidal drug.

6. The conjugate or pharmaceutical composition according to claim 5, wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5-dichloro-3-(4-methyl-1-piperazinyl)-6-[2-(1-piperidinyl)-1,3-thiazol-5-yl]benzo-1,4-quinone, Nutlin-3, MI-63, and any derivative thereof.

7. The conjugate according to any one of claims 1, 2, 5 or 6 or the pharmaceutical composition according to any one of claims 4 to 6 for use in treatment of mitochondrial disease.

8. The conjugate or pharmaceutical composition for use according to claim 7, wherein the mitochondrial disease is selected from the group consisting of chronic progressive external ophthalmoplegia, myoclonic epilepsy and ragged-red fiber disease, mitochondrial encephalomyopathy, lactic acidosis, and stroke-like episodes (MELAS), and Leigh syndrome.

9. The conjugate according to any one of claims 1, 2, 5 or 6 or the pharmaceutical composition according to any one of claims 4 to 6 for use in treatment of cancer.

10. The conjugate or pharmaceutical composition for use according to claim 9, wherein the cancer is selected from the group consisting of pancreatic cancer, colorectal cancer, thyroid gland cancer, lung cancer, head and neck cancer, uterine cervical cancer, endometrial cancer, ovary cancer, myelodysplastic syndrome, adenocarcinoma of the colon, and neuroblastoma.

11. A product which is:
(a) a kit comprising a conjugate according to either claim 1 or claim 2;
(b) a research reagent kit comprising a conjugate according to either claim 1 or claim 2;
(c) a kit for treatment comprising a conjugate according to either claim 1 or claim 2; or
(d) a kit for diagnosis comprising a conjugate according to either claim 1 or claim 2.

12. A kit comprising a pharmaceutical composition according to claim 4 and a cytocidal drug in combination.

13. The kit according to claim 12, wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5-dichloro-3-(4-methyl-1-piperazinyl)-6-[2-(1-piperidinyl)-1,3-thiazol-5-yl]benzo-1,4-quinone, and any derivative thereof.

## Patentansprüche

1. Konjugat einer DNA-bindenden Verbindung, die spezifisch an eine Sequenz von Doppelmembran-Organellen-DNA bindet, und einer Doppelmembran-Organellenlokalisierbaren Verbindung, wobei das Konjugat eine Struktur aufweist, die durch die folgende Formel (X), (XXII) oder (XXIII) dargestellt wird: 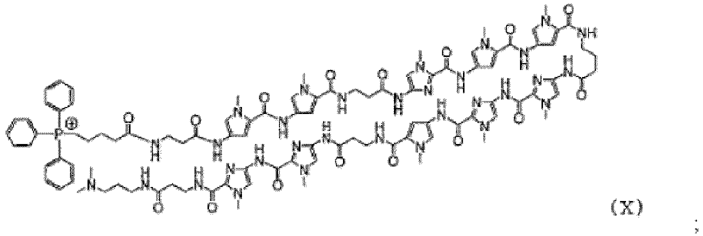 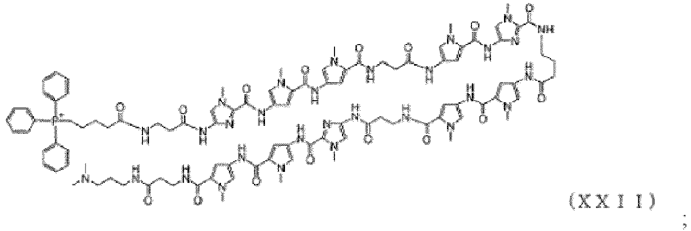 oder

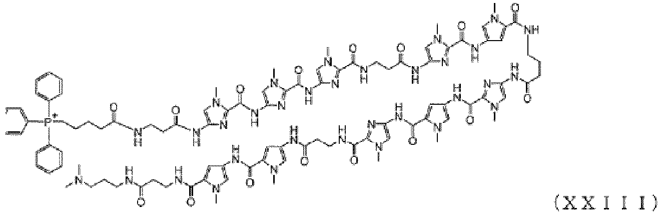

2. Konjugat einer DNA-bindenden Verbindung, die spezifisch an eine Sequenz von Doppelmembran-Organellen-DNA bindet, und einer Doppelmembran-Organellenlokalisierbaren Verbindung, wobei die DNA-bindende Verbindung eine Struktur aufweist, die durch die folgende Formel (VII) dargestellt wird, und die Doppelmembran-Organellenlokalisierbare Verbindung TPP (Triphenylphophonium) ist, wobei die Verbindung der Formel (VII) und das TPP direkt oder *über* einen Linker angelagert sind:

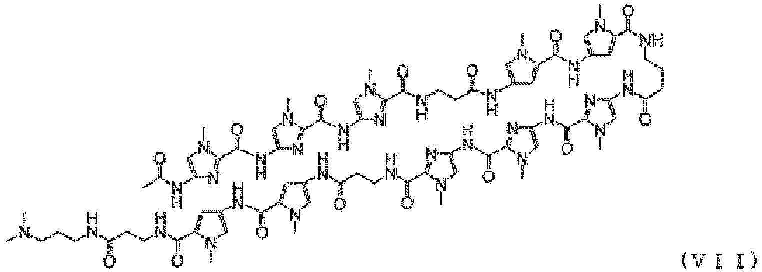

3. Zusammensetzung, die an eine mit einer mitochondrialen Erkrankung verwandte mitochondriale DNA-Sequenz bindet, umfassend ein Konjugat nach Anspruch 1 oder Anspruch 2.

4. Pharmazeutische Zusammensetzung, umfassend ein Konjugat nach Anspruch 1 oder Anspruch 2.

5. Konjugat nach Anspruch 1 oder 2 oder pharmazeutische Zusammensetzung nach Anspruch 5, die ferner mit einem zytotoxischen Arzneimittel kombiniert ist.

6. Konjugat oder pharmazeutische Zusammensetzung nach Anspruch 5, wobei das zytotoxische Arzneimittel aus der Gruppe ausgewählt ist, die aus Canagliflozin, Canagliflozin, Ipragliflozin, Dapagliflozin, Luseogliflozin, Tofogliflozin, Sergliflozin-Etabonat, Remogliflozin-Etabonat, Ertugliflozin, Sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycin, Piperlongumin, Hyperosid (Quercetin-3-galactosid), 2,3,5-Trichlor-6-(2-piperidin-1-yl-1,3-thiazol-5-yl)cyclohexa-2,5-dien-1,4-dion, 2,5-Dichlor-3-(1-piperidinyl)-6-[2-(1-piperidinyl)-1,3-thiazol-5-yl]benzo-1,4-chinon, 2,5-Dichlor-3-morpholin-4-yl-6-(2-piperidin-1-yl-1,3-thiazol-5-yl)cyclohexa-2,5-dien-1,4-dion, 2,5-Dichlor-3-(phenylamino)-6-(2-piperidin-1-yl-1,3-thiazol-5-yl)cyclohexa-2,5-dien-1,4-dion, 2,5-Dichlor-3-(2-morpholin-4-yl-1,3-thiazol-5-yl)-6-piperidin-1-ylcyclohexa-2,5-dien-1,4-dion, Obatoclax, Venetoclax, 2,5-Dichlor-3-(4-methyl-1-piperazinyl)-6-[2-(1-piperidinyl)-1,3-thiazol-5-yl]benzo-1,4-quinon, Nutlin-3, MI-63 und einem beliebigen Derivat davon besteht.

7. Konjugat nach einem der Ansprüche 1, 2, 5 oder 6 oder pharmazeutische Zusammensetzung nach einem der Ansprüche 4 bis 6 für die Verwendung bei der Behandlung einer mitochondrialen Erkrankung.

8. Konjugat oder pharmazeutische Zusammensetzung für die Verwendung nach Anspruch 7, wobei die mitochondriale Erkrankung aus der Gruppe ausgewählt ist, bestehend aus chronisch-progressiver externer Ophthalmoplegie, myoklonischer Epilepsie und Ragged-Red-Faser-Krankheit, mitochondrialer Enzephalomyopathie, Laktatazidose und Anfall ähnliche Episoden (MELAS) und Leigh-Syndrom.

9. Konjugat nach einem der Ansprüche 1, 2, 5 oder 6 oder pharmazeutische Zusammensetzung nach einem der Ansprüche 4 bis 6 für die Verwendung bei der Behandlung von Krebs.

10. Konjugat oder pharmazeutische Zusammensetzung für die Verwendung nach Anspruch 9, wobei der Krebs aus der Gruppe ausgewählt ist, die aus Bauchspeicheldrüsenkrebs, Darmkrebs, Schilddrüsenkrebs, Lungenkrebs, Kopf- und Halskrebs, Gebärmutterhalskrebs, Gebärmutterschleimhautkrebs, Eierstockkrebs, myelodysplastischem Syndrom, Adenokarzinom des Dickdarms und Neuroblastom besteht.

11. Produkt, das
(a) einen Kit, umfassend ein Konjugat nach Anspruch 1 oder Anspruch 2;
(b) einen Forschungsreagenzienkit, umfassend ein Konjugat nach Anspruch 1 oder Anspruch 2;
(c) einen Kit für die Behandlung, umfassend ein Konjugat nach Anspruch 1 oder Anspruch 2; oder
(d) einen Kit für die Diagnose, umfassend ein Konjugat nach Anspruch 1 oder Anspruch 2.

12. Kit, umfassend eine pharmazeutische Zusammensetzung nach Anspruch 4 und ein zytotoxisches Arzneimittel in Kombination.

13. Kit nach Anspruch 12, wobei das zytotoxische Arzneimittel aus der Gruppe ausgewählt ist, die aus Canagliflozin, Canagliflozin, Ipragliflozin, Dapagliflozin, Luseogliflozin, Tofogliflozin, Sergliflozin-Etabonat, Remogliflozin-Etabonat, Ertugliflozin, Sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycin, Piperlongumin, Hyperosid (Quercetin-3-galactosid), 2,3,5-Trichlor-6-(2-piperidin-1-yl-1,3-thiazol-5-yl)cyclohexa-2,5-dien-1,4-dion, 2,5-Dichlor-3-(1-piperidinyl)-6-[2-(1-piperidinyl)-1,3-thiazol-5-yl]benzo-1,4-chinon, 2,5-Dichlor-3-morpholin-4-yl-6-(2-piperidin-1-yl-1,3-thiazol-5-yl)cyclohexa-2,5-dien-1,4-dion, 2,5-Dichlor-3-(phenylamino)-6-(2-piperidin-1-yl-1,3-thiazol-5-yl)cyclohexa-2,5-dien-1,4-dion, 2,5-Dichlor-3-(2-morpholin-4-yl-1,3-thiazol-5-yl)-6-piperidin-1-ylcyclohexa-2,5-dien-1,4-dion, Obatoclax, Venetoclax, 2,5-Dichlor-3-(4-methyl-1-piperazinyl)-6-[2-(1-piperidinyl)-1,3-thiazol-5-yl]benzo-1,4-quinon und einem beliebigen Derivat davon besteht.

## Revendications

1. Conjugué d'un composé de liaison à l'ADN, qui se lie spécifiquement à une séquence d'ADN d'organite à double membrane, et d'un composé localisable dans des organites à double membrane, ledit conjugué ayant une structure représentée par les formules (X), (XXII) ou (XXIII) suivantes : 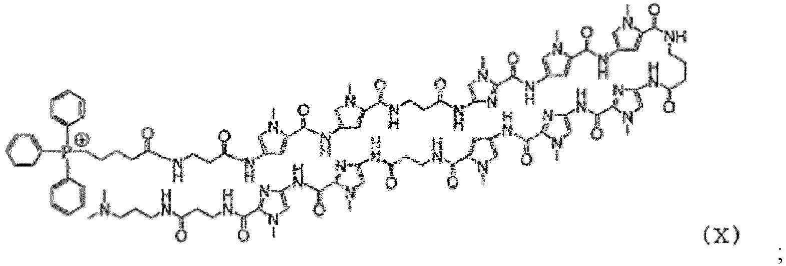 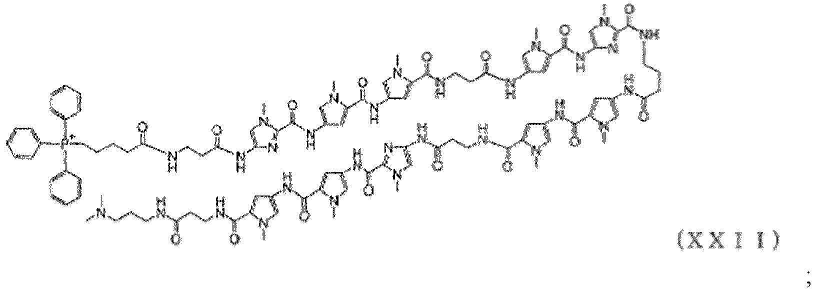 ou

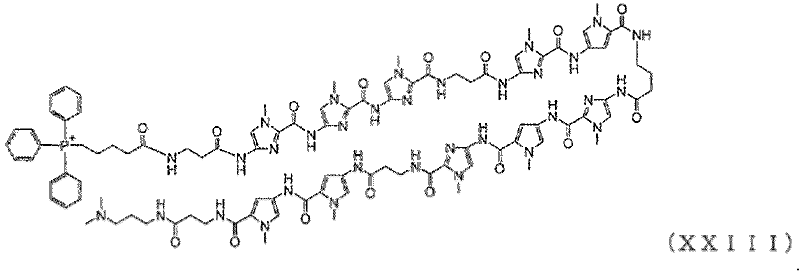

2. Conjugué d'un composé de liaison à l'ADN, qui se lie spécifiquement a une séquence d'ADN d'organite à double membrane, et d'un composé localisable dans des organites à double membrane, ledit composé de liaison à l'ADN ayant une structure représentée par la formule (VII) suivante et ledit composé localisé dans des organites à double membrane étant le TPP (triphénylphosphonium), ledit composé de la formule (VII) et ledit TPP étant liés directement ou par le biais d'un lieur :

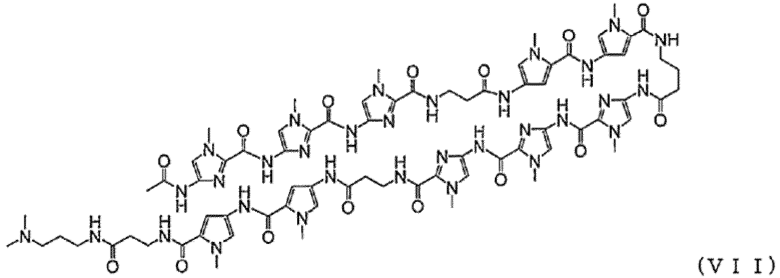

3. Composition qui se lie à une séquence d'ADN mitochondrial liée à une maladie mitochondriale, comprenant un conjugué selon la revendication 1 ou la revendication 2.

4. Composition pharmaceutique comprenant un conjugué selon la revendication 1 ou la revendication 2.

5. Conjugué selon la revendication 1 ou la revendication 2, ou composition pharmaceutique selon la revendication 4, en combinaison en outre avec un médicament cytocide.

6. Conjugué ou composition pharmaceutique selon la revendication 5, dans lesquels le médicament cytocide est choisi dans le groupe constitué par la canagliflozine, la canagliflozine, l'ipragliflozine, la dapagliflozine, la luséogliflozine, la tofogliflozine, l'étabonate de sergliflozine, l'étabonate de rémogliflozine, l'ertugliflozine, la sotagliflozine, le dasatinib, la quercétine, le navitoclax (ABT-263), l'ABT-737, l'A1331852, l'A1155463, la 17-(allylamino)-17-déméthoxygeldanamycine, la fisétine, le panobinostat, l'azithromycine, la roxithromicine, la piperlongumine, l'hyperoside (quercétine 3-galactoside), la 2,3,5-tricholoro-6-(2-pipéridin-1-yl-1,3-thiazol-5-yl)cyclohexa-2,5-diène-1,4-dione, la 2,5-dichloro-3-(1-pipéridinyl)-6-[2-(1-pipéridinyl)-1,3-thiazol-5-yl]benzo-1,4-quinone, la 2,5-dichloro-3-morpholin-4-yl-6-(2-pipéridin-1-yl-1,3-thiazol-5-yl)cyclohexa-2,5-diène-1,4-dione, la 2,5-dichloro-3-(phénylamino)-6-(2-pipéridin-1-yl-1,3-thiazol-5-yl)cyclohexa-2,5-diène-1,4-dione, la 2,5-dichloro-3-(2-morpholin-4-yl-1,3-thiazol-5-yl)-6-(pipéridin-1-yl)cyclohexa-2,5-diène-1,4-dione, l'obatoclax, le vénétoclax, la 2,5-dichloro-3-(4-méthylpipérazin-1-yl)-6-[2-(pipéridin-1-yl)-1,3-thiazol-5-yl]benzo-1,4-quinone, la nutline-3, le MI-63 et tout dérivé de ceux-ci.

7. Conjugué selon l'une quelconque des revendications 1, 2, 5 ou 6 ou composition pharmaceutique selon l'une quelconque des revendications 4 à 6 pour utilisation dans le traitement d'une maladie mitochondriale.

8. Conjugué ou composition pharmaceutique pour utilisation selon la revendication 7, dans lesquels la maladie mitochondriale est choisie dans le groupe constitué par l'ophtalmoplégie externe progressive chronique, le syndrome MERRF (myoclonic epilepsy and ragged-red fiber), le syndrome MELAS (mitochondrial encephalomyopathy, lactic acidosis and stroke-like episodes) et le syndrome de Leigh.

9. Conjugué selon l'une quelconque des revendications 1, 2, 5 ou 6 ou composition pharmaceutique selon l'une quelconque des revendications 4 à 6 pour utilisation dans le traitement du cancer.

10. Conjugué ou composition pharmaceutique pour utilisation selon la revendication 9, dans lesquels le cancer est choisi dans le groupe constitué par le cancer du pancréas, le cancer colorectal, le cancer de la thyroïde, le cancer des poumons, le cancer de la tête et du cou, le cancer du col de l'utérus, le cancer de l'endomètre, le cancer de l'ovaire, le syndrome myélodysplastique, l'adénocarcinome du colon et le neuroblastome.

11. Produit consistant en :
(a) un kit comprenant un conjugué selon la revendication 1 ou la revendication 2 ;
(b) un kit de réactifs de recherche comprenant un conjugué selon la revendication 1 ou la revendication 2 ;
(c) un kit de traitement comprenant un conjugué selon la revendication 1 ou la revendication 2 ; ou
(d) un kit de diagnostique comprenant un conjugué selon la revendication 1 ou la revendication 2.

12. Kit comprenant une composition pharmaceutique selon la revendication 4 et un médicament cytocide combinés.

13. Kit selon la revendication 12, dans lequel le médicament cytocide est choisi dans le groupe constitué par la canagliflozine, la canagliflozine, l'ipragliflozine, la dapagliflozine, la luséogliflozine, la tofogliflozine, l'étabonate de sergliflozine, l'étabonate de rémogliflozine, l'ertugliflozine, la sotagliflozine, le dasatinib, la quercétine, le navitoclax (ABT-263), l'ABT-737, l'A1331852, l'A1155463, la 17-(allylamino)-17-déméthoxygeldanamycine, la fisétine, le panobinostat, l'azithromycine, la roxithromicine, la piperlongumine, l'hyperoside (quercétine 3-galactoside), la 2,3,5-tricholoro-6-(2-pipéridin-1-yl-1,3-thiazol-5-yl)cyclohexa-2,5-diène-1,4-dione, la 2,5-dichloro-3-(1-pipéridinyl)-6-[2-(1-pipéridinyl)-1,3-thiazol-5-yl]benzo-1,4-quinone, la 2,5-cichloro-3-morpholin-4-yl-6-(2-pipéridin-1-yl-1,3-thiazol-5-yl)cyclohexa-2,5-diène-1,4-dione, la 2,5-dichloro-3-(phénylamino)-6-(2-pipéridin-1-yl-1,3-thiazol-5-yl)cyclohexa-2,5-diène-1,4-dione, la 2,5-dichloro-3-(2-morpholin-4-yl-1,3-thiazol-5-yl)-6-(pipéridin-1-yl)cyclohexa-2,5-diène-1,4-dione, l'obatoclax, le vénétoclax, le 2,5-dichloro-3-(4-méthylpipérazin-1-yl)-6-[2-(pipéridin-1-yl)-1,3-thiazol-5-yl]benzo-1,4-quinone, la nutline-3, le MI-63 et tout dérivé de ceux-ci.
